(19) Europäisches Patentamt
European Patent Office
Office européen des brevets

(11) Publication number: 0 127 897
B1

# (12) EUROPEAN PATENT SPECIFICATION

(45) Date of publication of patent specification: 27.12.89

(51) Int. Cl.$^4$: G 03 C 5/54, C 07 D 521/00

(21) Application number: 84106328.2

(22) Date of filing: 02.06.84

## (54) Photographic products and processes.

(30) Priority: 02.06.83 US 500366
02.06.83 US 500414
02.06.83 US 500415
02.06.83 US 500391

(43) Date of publication of application:
12.12.84 Bulletin 84/50

(45) Publication of the grant of the patent:
27.12.89 Bulletin 89/52

(84) Designated Contracting States:
DE FR GB NL

(56) References cited:
EP-A-0 009 989
GB-A-2 010 818
US-A-4 060 417
US-A-4 098 783

(73) Proprietor: POLAROID CORPORATION
549 Technology Square
Cambridge Massachusetts 02139 (US)

(72) Inventor: Rogers, Howard G.
20 Newton Street
Weston Mass. (US)
Inventor: Meneghini, Frank
123 Claremont Avenue
Arlington Mass. (US)
Inventor: Palumbo, Paul S.
46 Parsons Street
West Newton Mass. (US)
Inventor: Foley, James W.
40 Juniper Road
Andover Mass. (US)
Inventor: Arbree, Roberta R.
164 Coburn Woods
Nashua New Hampshire (US)

(74) Representative: Reitzner, Bruno, Dr. et al
Patentanwälte Dipl.-Ing. R. Splanemann Dr. B. Reitzner Tal 13
D-8000 München 2 (DE)

EP 0 127 897 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

1. Field of the Invention

This invention relates to the formation of dye images from a substantially colorless precursor of a preformed image dye. In another aspect, this invention relates to photographic products and a process for providing an imagewise distribution of a reagent such as a photographically active reagent or an image dye-providing moiety and to novel compounds useful therein.

2. Description of the Prior Art

US—A—3,719,489 describes and claims photographic processes employing initially photographically inert compounds which are capable of undergoing cleavage in the presence of the imagewise distribution of silver ions made available during processing of a silver halide emulsion to liberate a reagent, such as, a photographically active reagent or a dye in an imagewise distribution corresponding to that of said silver ions. In one embodiment disclosed therein, color images are produced by using as the photographically inert compounds, color providing compounds which are substantially non-diffusible in the photographic processing composition but capable of undergoing cleavage in the presence of the imagewise distribution of silver ions and/or soluble silver complex made available in the undeveloped and partially developed areas of a silver halide emulsion as a function of development to liberate a more mobile and diffusible color-providing moiety in an imagewise distribution corresponding to the imagewise distribution of said ions and/or said complex. The subsequent formation of a color image is the result of the differential in diffusibility between the parent compound and liberated color-providing moiety whereby the imagewise distribution of the more diffusible color-providing moiety released in the undeveloped and partially developed areas is free to transfer.

Compounds disclosed as useful in liberating a reagent in the presence of said silver ions and/or silver complex are sulfur-nitrogen compounds containing the group

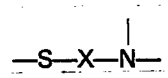

or —S—X—N= wherein X is

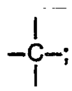 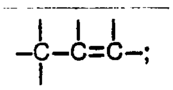 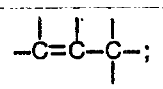 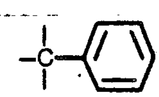 or 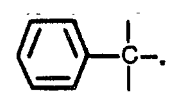

These 1,3-sulfur-nitrogen compounds may be linear or cyclic in structure, and in a particularly preferred embodiment are cyclic compounds, such as, thiazolidine compounds which comprise a dye radical having the chromophoric system of an azo, anthraquinone, phthalocyanine or other dye and a thiazolidin-2′-yl moiety which may be bonded directly to the dye radical or indirectly through an appropriate linking group.

US—A—4 060 417 describes and claims a photographic product which comprises a photosensitive element comprising at least two selectively sensitized silver halide emulsion layers having associated therewith a color-providing compound capable of undergoing cleavage in the presence of silver ions and/or soluble silver complex to liberate a color-providing moiety, one of said color-providing compound and said color-providing moiety being diffusible and the other being substantially non-diffusible in aqueous alkaline solution. The color-providing compounds contain a 1,3-sulfur-nitrogen group, but the formation of color in an imagewise distribution relies upon a differential in diffusibility between the color-providing compound (for example, a colorless precursor) and the color-providing moiety (for example, a complete image-dye). The color-providing compound is non-diffusible and upon silver ion assisted cleavage of the 1,3-sulfur-nitrogen group, a color-providing moiety is released which is diffusible. While the 1,3-sulfur-nitrogen group is used to provide this difference in diffusibility, it is not used to color-shift a color providing moiety preformed image-dye). The colorless or color-shifted color providing moiety (preformed dye) disclosed undergoes coloration or a color change non-imagewise due to an environmental change such as a pH change, by the removal of acyl groups via alkaline hydrolysis or by complexation of certain metal complexible dyes.

The present invention is concerned with another method of forming a color image.

Summary of the Invention

According to the present invention, the ability of 1,3-sulfur-nitrogen compounds to undergo silver ion assisted cleavage in an imagewise fashion is utilized to provide an imagewise distribution of a colored image dye from a substantially colorless precursor of a preformed image dye by employing a moiety comprising a 1,3-sulfur-nitrogen group to maintain said precursor in its substantially colorless form until said 1,3-sulfur-nitrogen group undergoes cleavage imagewise to correspond to the imagewise distribution of silver ion and/or soluble silver complex formed as a function of development of an imagewise exposed photosensitive element.

It is, therefore, the primary object of the present invention to provide a photographic product

comprising a support layer carrying, on one side thereof, a light-sensitive silver halide layer and a colorless precursor M—Z of a preformed image dye, having a moiety M and a radical Z, said moiety M comprising a 1,3-sulfur-nitrogen group A possessing a carbon atom in the 2-position intermediate said sulfur and nitrogen atoms, and undergoing cleavage in the presence of silver ions and/or soluble silver complex; said radical Z being the radical of the colorless precursor of a pre-formed image dye; such product is characterized in that said moiety M is substituted on said radical Z such that said precursor M—Z is maintained in its colorless form until said 1,3-sulfur-nitrogen group A undergoes cleavage.

Such cleavage occurs imagewise to correspond to the image distribution of silver ion and/or soluble silver complex made available as a function of development.

The present invention is concerned with a photographic product as defined above, to be used in a photographic color process which provides a dye image, said process comprising photo-exposing the photographic product including a silver halide emulsion having associated therewith a colorless precursor of a preformed image dye; and developing said exposed silver halide emulsion to form, as a function of development, a color image in dye from the above-defined colorless precursor. Preferably, the silver halide emulsion is a negative working emulsion and the color image is a positive image in dye.

The colorless image dye-providing compounds that may be employed in the above product may be represented by the formula

(I) M—Z

wherein M is a moiety comprising a 1,3-sulfur-nitrogen group A possessing a carbon atom in the 2-position intermediate said sulfur and nitrogen atoms, and undergoing cleavage in the presence of silver ions and/or soluble silver complex, and Z is the radical of a colorless precursor of a preformed image dye, characterized in that said M moiety is substituted on said radical Z such that said colorless precursor is maintained in its colorless form until said 1,3-sulfur-nitrogen group A undergoes cleavage.

The color image may be formed by using the imagewise cleavage of the 1,3-sulfur-nitrogen group to provide the image dye directly, or the imagewise cleavage of the 1,3-sulfur-nitrogen group may be used o activate a subsequent reaction or series of reactions which in turn provides the image dye. The silver ion assisted cleavage reaction together with any subsequent reaction or series of reactions should, of course, provide the image dye at a photographically useful rate in a given photographic system.

The 1,3-sulfur-nitrogen group may be derived from the various compounds disclosed in aforementioned US—A—3,719,489 including both the linear and cyclic compounds containing the grouping

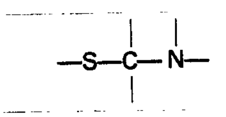 or 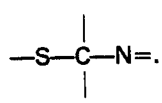.

Particularly preferred are the cyclic compounds where both the S and N atoms are included in the ring and especially the cyclic compounds illustrated in the following formula

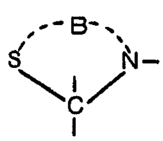

wherein B represents the atoms, preferably carbon atoms, necessary to complete a ring system containing at least 4 members and usually up to 20 members. Examples of such compounds include thiazolidines, benzothiazolines and 1,2-tetrahydrothiazines.

The image dye release from the colorless precursor compounds may comprise any of the general classes of dyes known in the art, for example, nitro, azo, xanthene and anthraquinone dyes; also leuco, indicator, temporarily "color shifted" and other dyes that take on a color change during or subsequent to processing to provide the ultimately desired color for the dye image via oxidation, changes in pH or alkaline hydrolysis of a blocking group. It will be appreciated that in the present invention such a color change is precluded without removal of the moiety, M, and also that said moiety and/or the radical of the colorless precursor of the preformed image dye may be substituted with solubilizing, ballasting or other groups as may be appropriate for a given photographic process.

One useful class of colorless precursors within the scope of formula (I) may be characterized as being a novel colorless precursor of a preformed image dye which is substituted with a moiety comprising a 1,3-sulfur-nitrogen group, said 1,3-sulfur-nitrogen group (a) being capable of undergoing cleavage in the presence of silver ion and/or soluble silver complex, and (b) possessing an amide substituent on the carbon atom in the 2-position that undergoes an intramolecularly accelerated cleavage reaction following the cleavage of said 1,3-sulfur-nitrogen group, which moiety maintains said precursor in its colorless form at least until the 1,3-sulfur nitrogen group undergoes said cleavage.

The novel colorless precursors M—2 may be represented by the formula:

(II) $$A—L—Z^1$$

wherein A is a 1,3-sulfur-nitrogen group capable of undergoing cleavage in the presence of silver ion and/or soluble silver complex; L is an amide moiety capable of undergoing an intramolecularly accelerated cleavage reaction following the cleavage of aid 1,3-sulfur-nitrogen group; and $Z^1$ is a preformed image dye rest which when taken with L is the colorless precursor radical Z of said preformed image dye, said A being substituted on said colorless precursor such that the precursor is maintained in its colorless form at least until said 1,3-sulfur-nitrogen group undergoes said cleavage.

As noted above, L is an amide moiety substituted in the 2-position of said 1,3-sulfur-nitrogen group A that undergoes an intramolecularly accelerated cleavage reaction following the cleavage of said 1,3-sulfur-nitrogen group. As an illustration, a 1,3-sulfur-nitrogen group may be used to mask the α-keto group of an α-ketoamide, such as, a glyoxalamide to depress its hydrolysis rate and provide a masked compound that is substantially stable to alkaline hydrolysis until the 1,3-sulfur-nitrogen group undergoes cleavage in the presence of silver ion and/or soluble silver complex. A 1,3-sulfur-nitrogen group also may be used to mask the aldehyde group of a phthalaldehydamide to prevent the aldehyde group from assisting the hydrolysis of the neighboring carboxamido group until such times as the 1,3-suflur-nitrogen group undergoes said silver ion assisted cleavage. It will be appreciated that any amide moiety may be used provided that the cleavage rate for the amide moiety taken with the silver assisted cleavage rate for the 1,3-sulfur-nitrogen group provides the image dye at a photographically useful rate for a given photographic system.

All formulae showing a 5-membered herteroring containing a S and a N atom comprise — in spite of the missing H — a thiazolidine ring in this invention.

Preferred compounds within the scope of formula (II) may be represented by the formulae:

(III)

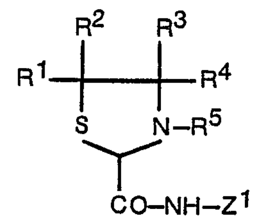

wherein $R^1$, $R^2$ and $R^3$ each are selected from hydrogen, alkyl, aryl, aralkyl and alkaryl; $R^4$ is selected from hydrogen, carboxy, N,N-dialkylcarboxyamido, alkyl, aryl, aralkyl and alkaryl; $R^5$ is selected from alkyl, aryl, alkaryl and aralkyl; and $Z^1$ is a preformed image dye rest which when taken with said —CO—NH— is the colorless precursor radical Z of a preformed image dye; and

(IV)

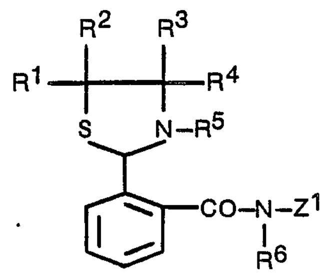

wherein $R^1$, $R^2$, $R^3$, $R^4$, and $R^5$ have the same meaning above; $R^6$ is alkyl or aryl; and $Z^1$ is a preformed image dye rest which when taken with said

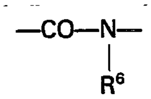

is a colorless precursor radical of a preformed image dye. $Z^1$ taken with said amide group to form said colorless precursor radical Z of a preformed image dye may be, for example, a nitroaniline dye radical of the formula:

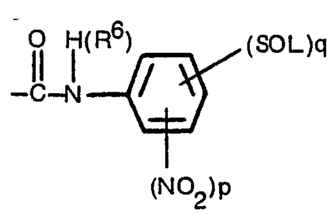

wherein $R^6$ has the same meaning given above, SOL is a solubilizing group, e.g., carboxy, hydroxy or sulfo; p is an integer 0 or 1; and q is an integer 0, 1 or 2; or an azo dye radical

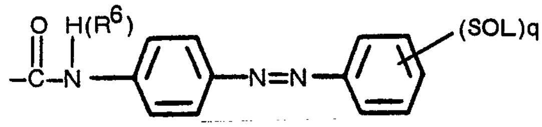

wherein $R^6$, SOL and q have the same meaning given above.

Typical aryl groups include phenyl and biphenyl and said alkyl groups comprising $R^1$, $R^2$, $R^3$, $R^4$, $R^5$ and $R^6$ usually contain 1 to 20 carbon atoms. Said aralkyl may be, for example, phenyl-substituted alkyl wherein said alkyl usually contains 1 to 20 carbon atoms, and said alkaryl may be, for example, alkyl-substituted phenyl wherein said alkyl usually contains 1 to 20 carbon atoms. The alkyl substituents of the N,N-dialkylcarboxamido group, the same or different, usually contain 1 to 6 carbon atoms.

The compounds of formulae III and IV can be synthesized using conventional methods. For example, the compounds of formula III may be prepared by reacting a 2-carboxy-thiazolidine with ethyl chloroformate and reacting the resulting intermediate with the selected aniline, azo or other compound to give the desired product. The compounds of formula IV can be prepared by reacting a thiazolidine-substituted benzoic acid with 1,1'-carbonyl-diimidazole to provide an intermediate which is reacted with the selected azo, aniline or other compound to give the desired product.

As examples of useful compounds within the scope of these formulae, mention may be made of:

(1)

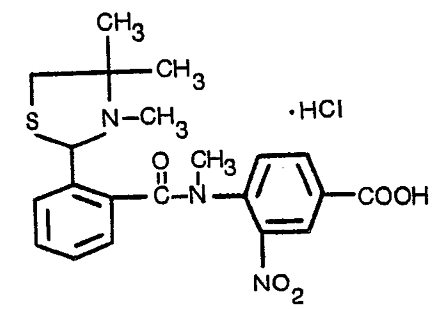

(2)

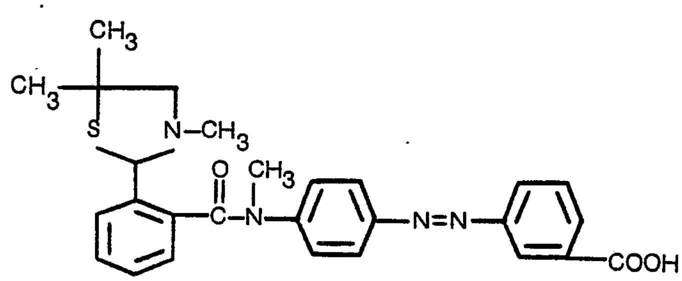

Another class of colorless precursors within the scope of formula (I) may be defined as a novel colorless precursor of a preformed image dye which is substituted with a moiety containing a thiazolidinyl group, said thiazolidinyl group (a) being capable of undergoing cleavage imagewise in the presence of an imagewise distribution of silver ion and/or soluble silver complex and (b) possessing a substituent on the carbon atom in the 2-position that undergoes a β-elimination reaction upon said imagewise cleavage, which moiety maintains the precursor in its colorless form at least until said thiazolidinyl group undergoes said cleavage.

These novel colorless precursors may be represented by the formula:

(V) $$T^1—L^1—Y—Z^1$$

wherein $T^1$ is a thiazolidin-2'-yl group capable of undergoing cleavage in the presence of silver ions and/or soluble silver complex; $L^1$ is a moiety capable of undergoing a beta-elimination reaction following the cleavage of said thiazolidin-2'-yl group; Y is a leaving group released by said beta-elimination reaction; and $Z^1$ is a preformed image dye rest which taken with Y is a colorless precursor radical Z of said preformed image dye, said $T^1—L^1—$ being substituted on said precursor such that the precursor is maintained in its colorless form until said thiazolidin-2'-yl group undergoes said cleavage.

β-elimination reactions are well known in the art and involve the breaking of bonds, for example, a C—N, C—O, C—S, C—Se, N—N, N—O or other bond to release a leaving group, which in this instance would comprise the image dye. Any moiety that undergoes β-elimination may be employed as $L^1$ in formula V above, provided that the elimination rate for the moiety taken with the silver assisted cleavage rate provides the image dye at a photographically useful rate in a given photographic system. The rate

constants for various leaving groups in elimination reactions of β-substituted sulphones, β-substituted phenyl ketones and β-substituted esters have been reported by Charles J. M. Stirling et al., J. Chem. Soc. (B), 1970, pages 672 and 684; Charles J. M. Stirling et al, J. Chem. Soc. Chem. Commun., page 941 (1975); and Charles J. M. Stirling, Acc. Chem. Res. 12, pages 198—203 (1979). Examples of some leaving groups from a carbon system include —SMe; —SPh; —SePh; —OPh; —OMe; $—P(O)(OEt)_2$; —NHTs; $—C(Me)_2NO_2$; —N(Me)Ts; —N(Me)Ac; —N(Ph)Ac; —N(Ph)Ts; $—N(Ph)CO_2CH_2Ph$ and $—N(Me)CO_2Ph$ wherein Me, Et, Ph, Ac and Ts represent methyl, ethyl, phenyl, acetyl and tosyl, respectively.

The preferred compounds of Formula (V) may be represented by the formula:

(VI)

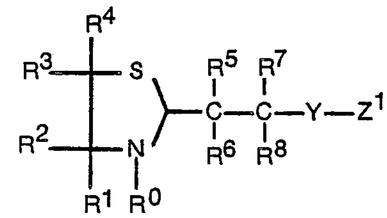

wherein $R^0$ is selected from alkyl, aryl, aralkyl and alkaryl; $R^1$ is selected from hydrogen, carboxy, N,N-dialkylcarboxamido, alkyl, aryl, aralkyl and alkaryl; $R^2$, $R^3$ and $R^4$ each are selected from hydrogen, alkyl, aryl, aralkyl and alkaryl; $R^5$ is selected from hydrogen and a group that can be removed upon cleavage of said thiazolidinyl group to leave an electron pair, e.g., a carboxy group; $R^6$, $R^7$ and $R^8$ each are selected from hydrogen, alkyl, aryl, aralkyl and alkaryl; and Y is a leaving group released by a beta-elimination reaction following cleavage of said thiazolidinyl group; and $Z^1$ is a preformed image dye rest which when taken with Y is a colorless precursor radical of a preformed image dye.

Particularly preferred compounds within the scope of formulae (V) and (VI) release the image dye as a carbamic acid and may be represented by the formula

(VII)

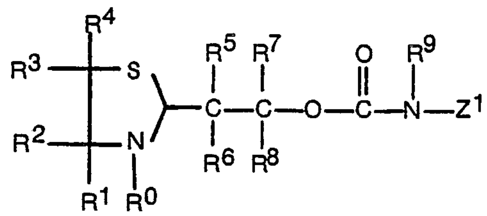

wherein $R^0$, $R^1$, $R^2$, $R^3$, $R^4$, $R^5$, $R^6$, $R^7$, $R^8$ have the same meaning given above; $R^9$ is hydrogen, alkyl, aryl, aralkyl or alkaryl; and $Z^1$ taken with

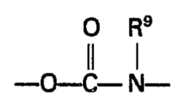

is a colorless precursor radical of a preformed image dye.

The moiety

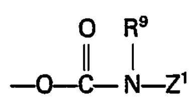

comprising the colorless precursor radical of a preformed image dye may be, for example,

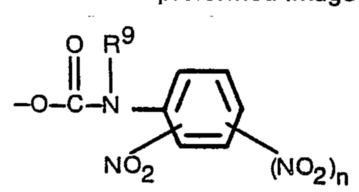

wherein $R^9$ has the same meaning given above and n is 0 or 1.

Typical aryl groups include phenyl and biphenyl and said alkyl groups comprising $R^0$, $R^1$, $R^2$, $R^3$, $R^4$, $R^5$, $R^6$, $R^7$, $R^8$ and $R^9$ usually contain 1 to 20 carbon atoms, such as methyl, ethyl, propyl, isopropyl, isobutyl, hexyl, dodecyl, octadecyl and eicosanyl. Said aralkyl may be, for example, phenyl-substituted alkyl wherein said alkyl usually contains 1 to 20 carbon atoms, and said alkaryl may be, for example, alkyl-substituted phenyl wherein said alkyl usually contains 1 to 20 carbon atoms. When $R^1$ is N,N-dialkylcarboxamido, each alkyl usually contains 1 to 20 carbon atoms.

It will be appreciated that the above-denoted $R^0$ to $R^9$ groups may be further substituted with, for example, amino, carboxy, hydroxy, alkoxy, sulfonamido or other appropriate group.

The compounds of formulae (V) to (VII) can be synthesized in a conventional manner, for example, by reacting a 2-substituted thaizolidine compound with another molecule comprising the selected reagent to give the desired product or by reacting an aminoethanethiol with an aldehyde-substituted molecule comprising the reagent to give the desired product.

As examples of such novel compounds, mention may be made of the following:

(3)

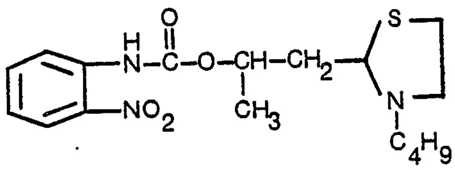

(4)

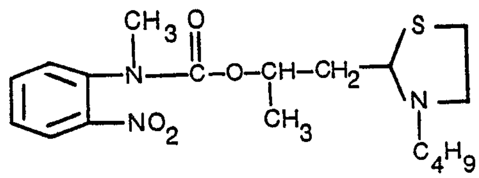

(5)

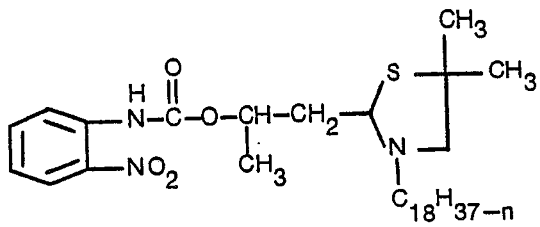

(6)

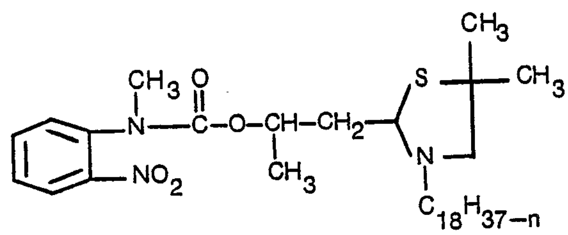

Yet another class of colorless precursors within the scope of formula (I) may be defined as a novel colorless precursor of a preformed image dye which is substituted with a moiety containing a thiazolidinyl group, said thiazolidinyl group (a) being capable of undergoing cleavage imagewise in the presence of an imagewise distribution of silver ion and/or soluble silver complex and (b) possessing a substituent on the carbon atom in the 2-position which upon cleavage of said thiazolidinyl group, undergoes a β-elimination reaction followed by an intramolecularly accelerated nucleophilic displacement reaction, which moiety maintains the precursor in its colorless form at least until said thiazolidinyl group undergoes said cleavage.

These novel precursors may be represented by the formula:

(VIII) $$T^1—L^1—Y^1—Z^1$$

wherein $T^1$ is a thiazolidin-2′-yl group capable of undergoing cleavage in the presence of silver ions and/or soluble silver complex; $L^1$ is a moiety capable of undergoing a beta-elimination reaction following cleavage of said thiazolidin-2′-yl group; $Y^1$ is a moiety capable of undergoing an intramolecularly accelerated nucleophilic displacement reaction following said beta-elimination reaction; and $Z^1$ is a preformed image dye rest which when taken with $Y^1$ is the colorless precursor radical Z of a preformed image dye, said $T^1—L^1—$ being substituted on said colorless precursor such that the precursor M—Z is maintained in its colorless form until said thiazolidin-2′-yl group undergoes said cleavage.

As mentioned previously, β-elimination reactions are well known in the art and involve the breaking of bonds, for example, a C—N, C—O, C—S, C—Se, N—N, N—O or other bond to release a leaving group, which in this instance would comprise $Y^1$ that ultimately would release the image dye by said nucleophilic displacement reaction. Any moiety that undergoes β-elimination may be employed as $L^1$ in formula VIII above, provided that the elimination rate and the rate of said nucleophilic displacement taken with the silver assisted cleavage rate provides the image dye at a photographically useful rate in a given photographic system.

Intramolecularly accelerated nucleophilic displacement reactions and various groups including, for example, esters and amides which undergo such reactions are well known in the art and have been described in US—A—3,980,479; 4,139,379; 4,199,354; and 4,199,355.

In a preferred embodiment of the presnet invention, the β-elimination reaction which occurs as a result of the cleavage of the thiazolidinyl group is used to generate a neighboring group which anchimerically assists the cleavage of an amide group and particularly a carboxamido group, i.e., the nucleophilic displacement reaction effects cleavage of an amide to provide the imagewise distribution of dye or other reagent. For example, said 2-substituent may include a carboxamido-phenyl moiety which is stable to cleavage until an anchimerically assisting group, such as, —$CH_2OH$ is made available ortho to the carboxamido by β-elimination whereupon the carboxamido group undergoes cleavage imagewise to correspond to the imagewise distribution of said —$CH_2OH$.

Preferred compounds of formula (VIII) may be represented by the formula

(IX)

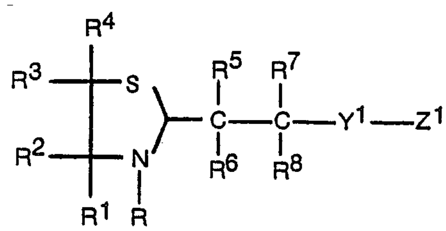

wherein R is selected from alkyl, aryl, aralkyl, and alkaryl; $R^1$ is selected from hydrogen, carboxy, N,N-dialkylcarboxamido, alkyl, aryl, aralkyl and alkaryl; $R^2$, $R^3$ and $R^4$ each are selected from hydrogen, alkyl, aryl, aralkyl and alkaryl; $R^5$ is selected from hydrogen and a group that can be removed upon cleavage of said thiazolidinyl group to leave an electron pair, e.g., a carboxy group; $R^6$, $R^7$ and $R^8$ each are selected from hydrogen, alkyl, aryl, aralkyl and alkaryl; $Y^1$ is a moiety released by a β-elimination reaction capable of undergoing an intramolecularly accelerated nucleophilic displacement reaction; and $Z^1$ taken with $Y^1$ is a colorless precursor radical of a preformed image dye.

Particularly preferred compounds may be represented by the formula

(X)

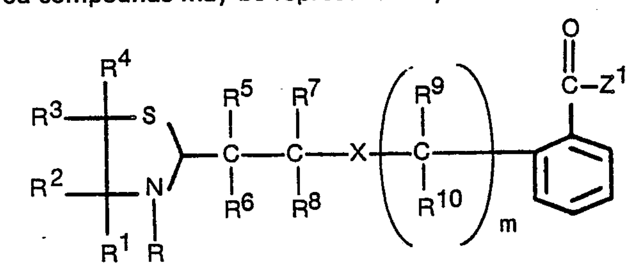

wherein R, $R^1$, $R^2$, $R^3$, $R^4$, $R^5$, $R^6$, $R^7$, and $R^8$ have the same meaning given above; $R^9$ and $R^{10}$ each are hydrogen, alkyl, aryl, aralkyl or alkaryl X is a group that will effect cleavage of said

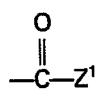

by intramolecular nucleophilic displacement; m is 0 or a positive integer capable of providing said intramolecular nucleophilic displacement at an accelerated rate; and $Z^1$ taken with

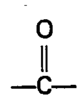

is the colorless precursor radical of a preformed image dye.

Where $Z^1$ taken with

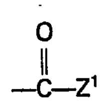

comprises the colorless precursor radical of a preformed image dye, $Z^1$ may be, for example,

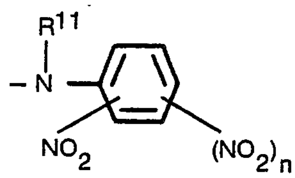

wherein $R^{11}$ is hydrogen, alkyl, aryl, alkaryl or aralkyl and n is 0 or 1.

Typical aryl groups include phenyl and biphenyl and said alkyl groups comprising R, $R^1$, $R^2$, $R^3$, $R^4$, $R^5$, $R^6$, $R^7$, $R^8$, $R^9$, $R^{10}$ and $R^{11}$ usually contain 1 to 20 carbon atoms, such as methyl, ethyl, propyl, isopropyl, isobutyl, hexyl, dodecyl, octadecyl and eicosanyl. Said aralkyl may be, for example, phenyl-substituted alkyl wherein said alkyl usually contains 1 to 20 carbon atoms, and said alkaryl may be, for example, alkyl-substituted phenyl wherein said alkyl usually contains 1 to 20 carbon atoms. When $R^1$ is N,N-dialkylcarboxamido, each alkyl usually contains 1 to 20 carbon atoms.

It will be appreciated that the above-denoted R groups may be further substituted with, for example, amino, carboxy, hydroxy, alkoxy, sulfonamido or other appropriate group.

Examples of X include

—O—, 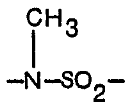 and 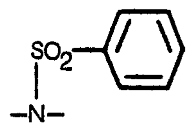

and typically, m is 0 or 1.

The novel compounds of formulae (VIII) to (X) can be synthesized in a conventional manner, for example, by reacting a 2-substituted thiazolidine compound with another molecular comprising the selected reagent to give the desired product or by reacting an aminoethanethiol with an aldehyde-substituted molecule comprising the selected reagent to give the desired product.

As examples of these novel compounds, mention may be made of the following:

(7) 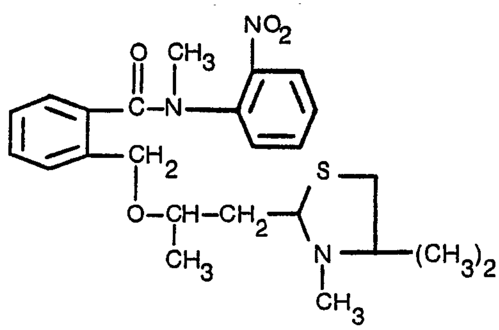

(8) 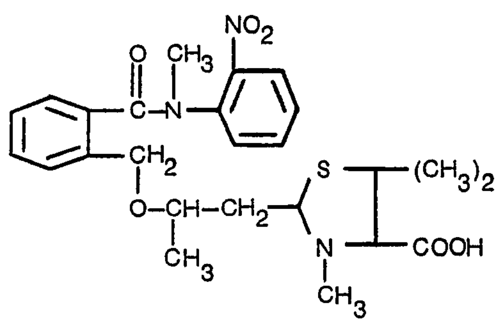

As noted above, the image dye released from the colorless precursor compounds may comprise any of the general classes of dyes known in the art, for example, nitro, azo, xanthene and anthraquinone dyes; also leuco, indicator, temporarily "color shifted" and other dyes that take on a color change during or subsequent to processing to provide the ultimately desired color for the dye image via oxidation, changes in pH or alkaline hydrolysis of a blocking group. In the present invention such a color change is precluded without removal of the moiety, M, comprising the 1,3-sulfur-nitrogen group.

The following examples are given to further illustrate the present invention and are not intended to be limiting.

Example 1

Preparation of the compound having the formula

(1) 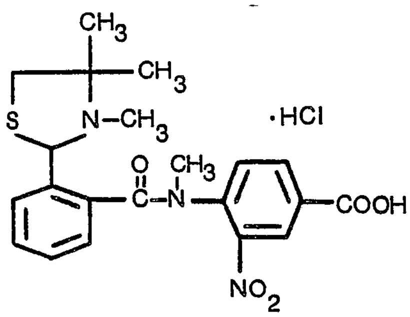

2-(2′-carboxyphenyl)-3,5,5-trimethyl-thiazolidine (6.00 g; 0.0239 mol) was mixed with 1,1′-carbonyldiimidazole (3.88 g; 0.0239 mol) in 70 ml of dry N,N-dimethylformamide (DMF). Carbon dioxide evolution was observed. After stirring for one hour at room temperature, 4-carboxy-2-nitro-N-methylaniline (4.69 g; 0.0239 mol) was added providing an orange solution. Portion-wise addition of sodium hydroxide (50% oil dispersion, 3.44 g; 0.0717 mol) over a twenty minute period was accompanied by hydrogen evolution and an exotherm (to 50°C). The resulting dark brown slurry was stirred at ambient temperature for fifteen minutes producing a dark brown gel. The mixture was kept overnight in the dark and under nitrogen. The mixture was acidified with dilute hydrochloric acid (14 ml conc. HCl in 130 ml of water), then adjusted to pH 4 with dilute aqueous sodium hydroxide. The mixture was filtered to give 2.7 g of an orange-yellow solid (the 2-nitro-4-carboxy-N-methylaniline reactant). The aqueous-DMF filtrate was saturated with sodium chloride and extracted with chloroform. A solid appeared (insoluble in both layers) which was filtered, washed with water and acetone. After drying, 1.96 g of the title compound was obtained as a cream powder, melting range 251.5—253.5°C (dec.). The dried (sodium sulfate) chloroform layer was evaporated to provide an orange oil which was triturated with 100 ml of ether. Solid appeared and was filtered off (1.57 g of the starting thiazolidine reactant). The ether filtrate was treated with HCl gas to pecipitate 2.24 g of the mixed hydrochlorides of the starting thiazolidine reactant and of the title compound. Separation was achieved by dissolving the mixture in 15 ml of boiling methanol. After carbon treatment and filtration, ether (100 ml) was added to the methanolic solution along with some seed crystals of the title compound. In this manner an additional 0.81 g of the title compound was realized; melting point 255°C (dec.); total yield 2.77 g (25% by weight).

Analysis for $C_{27}H_{24}ClN_3O_5S$:

| | | | | | | |
|---|---|---|---|---|---|---|
| Calculated: | C, 54.1; | H, 5.2; | Cl, 7.6; | N, 9.0; | O, 17.2; | S, 6.9 |
| Found: | C, 54.0; | H, 5.3; | Cl, 7.7; | N, 8.9; | O, 17.4; | S, 6.7 |

Example 2

Preparation of the compound having the formula

(2) 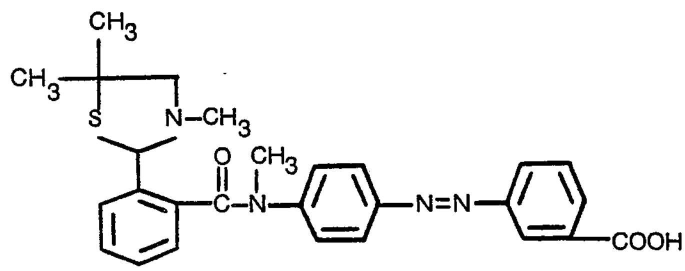

2.51 g of 2-(2′-carboxyphenyl)-3,5,5-trimethylthiazolidine and 1.62 g of 1,1′-carbonyldiimidazole were placed in a flask under a drying tube, and 15 ml of N,N-dimethylformamide were added at room temperature. The reaction mixture was stirred for 30 minutes giving the following intermediate designated A.

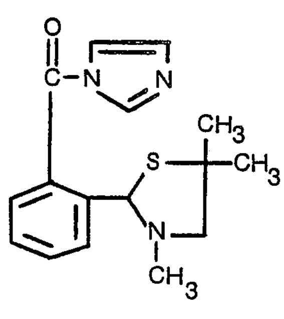 (A)

To this reaction mixture comprising the above intermediate was added 2.50 g of the azo compound designated B below.

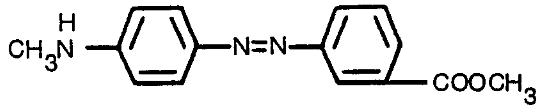

(B)

After stirring fifteen minutes, 0.50 g of a 50% oil dispersion of NaH was added in two portions. (The temperature rose slightly and foaming occurred.) The reaction mixture was stirred at room temperature for one hour and then at 60—70°C for two and one-half hours. After cooling to room temperature, the reaction mixture was poured into water, extractd 3x with ether and the ether extracts were washed with water, brine and dried. 200 mg of the following intermediate designated C were separated from the crude reaction mixture using preparative thin layer chromatography (TLC) techniques.

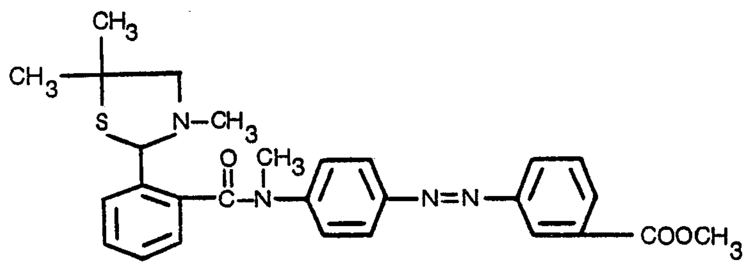

(C)

200 mg of C was dissolved in methanol, and 7 ml of water containing potassium hydroxide was added to the methanol solution. The resulting mixture was milky, and additional methanol (10 ml) was added with stirring until the solution became a clear yellow. The solution was then heated to 60—62°C with stirring. After TLC showed the ester hydrolysis reaction complete, the reaction mixture was placed under aspirator vacuum for 10 minutes at 60°C and then under a vacuum pump at 50°C. When most of the methanol had been removed, a yellow oil precipitated. The oil was dissolved in 30 ml of water and the solution extracted 3x with ether, adjusted to pH 5 and extracted 2x with ether. The ether extracts were combined, washed with brine and dried over sodium sulfate. The title compound was purified using preparative TLC techniques.

When a sample of the title compound was dissolved in 0.5 mol NaOH, the resulting solution appeared stable and visually to be almost colorless. Upon the addition of silver thiosulfate, a yellow color developed within 3 seconds, and after 15—30 seconds, the solution was an intense yellow.

The azo compound B employed in the above example was prepared as follows:

3-Aminobenzoic acid (27.4 g) was placed in 350 ml of aqueous 85% lactic acid and cooled to −10°C. Sodium nitrite (14.0 g) was dissolved in a small amount of water and added slowly to the amino acid-lactic acid solution so that the temperature remained below 0°C. After this addition was complete, stirring was continued for 30 minutes at −10°C and then at 0°C to +5°C for 15 minutes. The solution was then cooled to −10°C and N-methylaniline (30.0 g) in 200 ml of aqueous 85% lactic acid was slowly added. A light yellow precipitate formed. The reaction solution was stirred at 0°C for one hour and at room temperature for two hours. (The reaction mixture become red-brown.) It was then heated at 70°C for two hours and allowed to stand overnight. The reaction mixture was diluted to 2 liters with water and filtered to collect a brown solid. The brown solid comprising the crude reaction product was treated with one liter of aqueous sodium bicarbonate at room temperature and stirred. The aqueous solution was washed with ether, acidified to pH 5 and a small amount of red precipitate was isolated. A large amount of the brown crude reaction product did not dissolve so the undissolved cruder product was treated with sodium bicarbonate at 80°C. After filtering, extracting the filtrate with ether and acidifying to pH 5, an orange powder was obtained. The orange powder was dissolved in 500 ml of dry methanol. HCl gas was bubbled through the methanol solution for 20 minutes, and the solution was heated to 60°C for one hour then allowed to stand overnight. It was poured into 3.5 liters of water, and the solid collected was washed with water, stirred 3x with hot sodium bicarbonate, then dissolved in chloroform. The chloroform solution was washed with sodium bicarbonate, dried over sodium sulfate and evaporated under vacuum to give 9 g of the title compound as an orange-red oil that solidified after drying at 90°C under vacuum.

## Example 3

Preparation of the compound having the formula

(3)

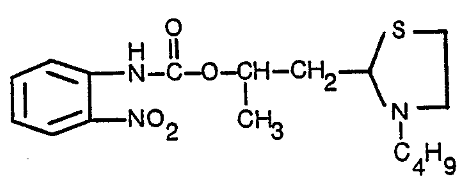

1 g of the following carbamoyl chloride-isocyanate mixture

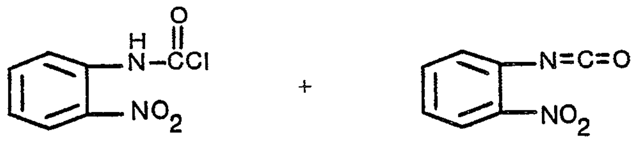

was added to 20 ml of dry chloroform. To this mixture was added 1 g of the thiazolidine alcohol of the formula

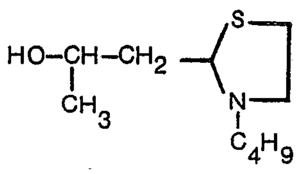

This reaction mixture was stirred at room temperature for two hours. Additional chloroform solvent was added so that any starting material present as the HCl salt would be soluble. TLC in ether was recorded. The reaction mixture was evaporated and triturated with ether. The insoluble solid was presumed to be the HCl salt of the title compound. TLC had shown a considerable amount of dark material which remained at the origin, so the solid was chromatographed on silica with chloroform as eluent. The amine and the title compound came off with the front as a mixture. TLC showed a minor amount of amine plus another colored impurity. NMR showed mostly title compound that came off as free base. The free base was soluble in ether and positions in NMR compared with those of the starting alcohol (free base).

The thiazolidine alcohol having the formula set out below was prepared as follows:

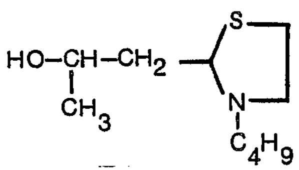

A mixture of 2-n-butylaminoethanethiol (4.5 g; 0.034 mol) and the dimer of β-hydroxy-n-butyraldehyde (3.0 g; 0.017 mol) in 100 ml of benzene were refluxed under nitrogen using a Dean-Stark trap to remove water. (Water began to come over immediately and stopped after 20 minutes.) Refluxing of the mixture was continued for one hour and then the benzene was removed by evaporation. The residue was distilled (boiling point 100°C at 0.125 mm) to give the title compound as a colorless oil.

## Example 4

Preparation of the compound having the formula

(4)

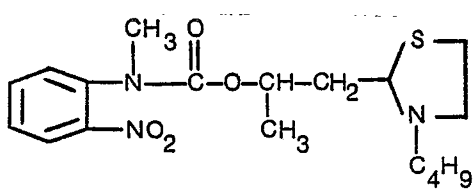

The compound of Example 3 (200 mg; 0.54 mmole) in 0.5 ml of anhydrous N,N-dimethylformamide (over 0.4 nm molecular sieves) was added to sodium hydride (13 mg; 0.54 mmole) in N,N-dimethylformamide under nitrogen. After stirring at room temperature for 1½ hours, evolution of hydrogen had ceased. Methyl iodide (77 mg; 0.54 mmole) dissolved in N,N-dimethylformamide was added dropwise. Heat evolved. Stirring of the reaction mixture was continued at room temperature. Sodium iodide precipitated. The solution of the carbamate anion was red. As the reaction with methyliodide proceeded, the color changed to orange. The time for complete color change was 1½ hours. Thereafter, the mixture was poured into water, extracted with ether, the ether extract washed several times with water, dried and evaporated. The yield of title compound was nearly quantitative.

Example 5

Preparation of the compound having the formula

(5)

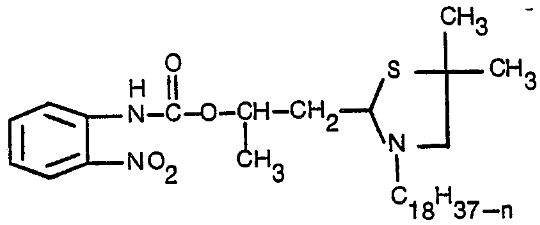

The thiazolidine alcohol of the formula,

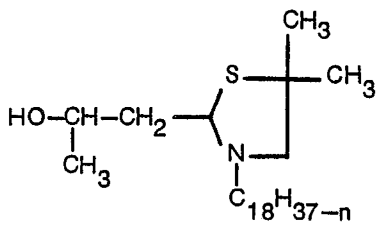

(1.85 g) was dissolved in 25 ml dry chloroform. Solid o-nitrophenyl isocyanate was added, and the reaction mixture was stirred over the weekend. It was then evaporated, and the oil residue was dissolved in petroleum ether and extracted five times with water. The petroleum ether layer was dried and evaporated. (NMR showed the desired product.) Some solid formed in the oil. It was insoluble in petroleum ether and was removed by dissolving the product in petroleum either and filtering. Evaporation of the petroleum either gave an orange oil which crystallized on standing. The crystals were slightly soluble in isopropanol. The partially crystalline oil was triturated with isopropanol, filtered and the crystals washed with isopropanol and dried to give 400 mg of the title compound as pale yellow crystals (melting range 53°—55°C).

Example 6

Preparation of the compound having the formula

(6)

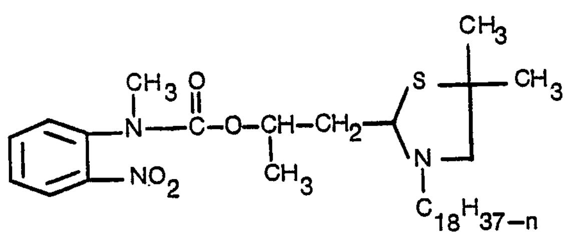

The compound of Example 6 was prepared from the compound of Example 5 (1.1 g; 1.86 mmol) according to the procedure given in Example 4 using equimolar amounts, i.e., 1.86 mmol of both sodium hydride and methyl iodide in N,N-dimethylformamide. Evaporation of the ether extract of the reaction mixture gave an orange oil. TLC showed the title compound as the major product (which changed to orange in several hours) plus a very minor amount of two yellow impurities.

Example 7

Preparation of the compound having the formula

(7)

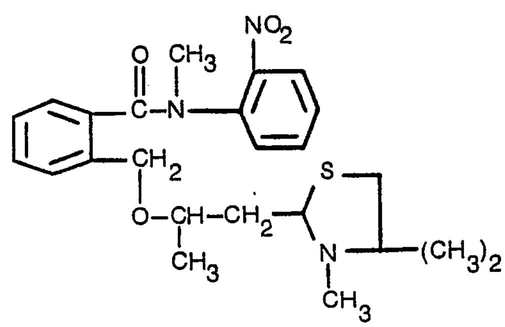

The compound of the formula

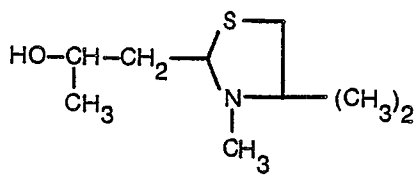

(2.3827 g; 0.0126 mol) was dissolved in 80 ml tetrahydrofuran ($Al_2O_3$ treated) and placed in a flamed out flask under argon. Potassium t-butoxide (1.695 g; 0.0151 mol) was added at room temperature and the resulting opaque, slightly orange solution was stirred for 15 minutes. Then the compound of the formula

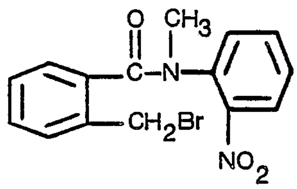

(4.395 g; 0.0126 mol) was added as a solid, and the resulting solution, which turned deep red in color, was stirred overnight. (TLC showed the desired product as well as both starting materials.) To this reaction mixture was added 3 ml of dry N,N-dimethylformamide followed by 0.7 g of potassium t-butoxide. The solution, which became darker in color, was poured into 150 ml water/250 ml ether, shaken, separated and the ether layer washed several times with water. The ether layer was then extracted with 1N HCl (3 × 150 ml). The acid extracts were combined and neutralized with solid sodium bicarbonate. The cloudy aqueous solution was extracted with chloroform (2 × 150 ml), dried over anhydrous sodium sulfate and evaporated to dryness to give the title compound.

Purified samples of the title compound were obtained by both 1) $SiO_2$ preparative plate chromatography and three chloroform elutions and 2) medium pressure liquid chromatography using chloroform as solvent.

Example 8

Preparation of the compound having the formula

(8)

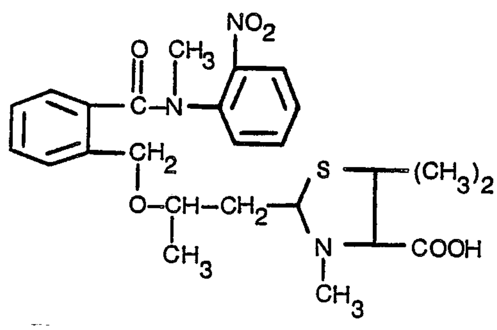

The compound of the formula,

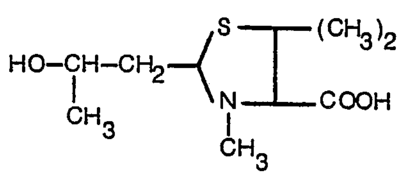

(0.1136 g; 0.487 nmol) was dissolved in 3 ml tetrahydrofuran (previously dried using $Al_2O_3$ and molecular sieves) and placed in a flamed out flask under argon in a 0°C bath. To this solution was added n-butyllithium in hexane (0.61 ml/0.974 nmol) over 5 seconds, and then the compound of the formula

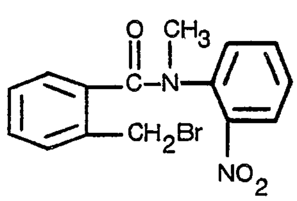

(0.170 g; 0.487 mmol) in 1 ml dry tetrahydrofuran also was added over 5 seconds. A pale yellow color was noted.

The reaction mixture was stirred at 0°C, and after 5 minutes the bath was removed. TLC after 0.5 hour at room temperature showed that product formation apparently was very slow at room temperature so the reaction mixture was heated to reflux for 2.5 hours. After cooling to room temperature, the mixture was poured into ether and filtered. The filtered material was dissolved in water and the pH adjusted to pH 4 in 1N HCl. The precipitate that formed was removed by filtration and the filtrate extracted two times with chloroform, dried over anhydrous sodium sulfate and evaporated to dryness to yield the title compound.

As noted previously, the present invention is concerned with the formation of a color image from certain colorless image dye-providing compounds comprising a colorless precursor of a preformed image dye. In this invention, the colorless compound may be present initially in the phorosensitive element in a layer or layers other than the layer containing the light-sensitive silver halide emulsion, or it may be in the light-sensitive layer itself. For example, it may be in a layer on one side of the emulsion or in two layers, one on either side of the emulsion. If desired, it may be separated from the emulsion layer by one or more spacer layers. Where the colorless compound is present in the light-sensitive layer, the compound should be inert, that is photographically innocuous in that it does not adversely effect or impair image formation. If not photographically innocuous, the compound may be modified in a manner which does not interfere with the development process in any way, but which deactivates the compound so that it does not affect adversely the light-sensitive emulsion. Rather than being disposed in the photosensitive element, the colorless compound may be initially contained in a layer associated with an image-receiving layer in processes such as diffusion transfer processes where image-receiving elements are employed.

The formation of color images according to the subject invention is applicable to the preparation of both monochromatic and multicolor images. Four example, the colorless image dye-providing compounds of this invention may be employed in photographic systems utilizing multilayer photosensitive elements comprising at least two selectively sensitive silver halide emulsion strata having said colorless image dye-providing compounds associated therewith which are processed simultaneously and without separation to provide a multicolor image. In such a structure, a barrier interlayer of silver complex scavenger, e.g., silver precipitant may be used, to confirm diffusion of soluble silver complex to the appropriate stratum. Also, filter layers containing, e.g., bleachable filter dyes of the type described in US—A—4,304,833, 4,358,118 and 4,304,834 may be used to control the spectral composition of light falling on the underlying light-sensitive layer. Another useful structure for obtaining multicolor images is the screen type negative described in US—A—2,968,554 or that described in US—A—3,019,124.

According to one method of forming color images, both the image dyes and their colorless parent compounds comprising the colorless precursor of a preformed image dye are substantially non-diffisible from their initial position in association with the photosensitive strata. To achieve the requisite non-diffusibility, the colorless parent compound may be appropriately substituted with an immobilizing group, e.g., a long chain alkyl group and the image dye released may be a dye that is substantially non-diffusible by nature or it may be rendered non-diffusible by appropriate substitution with an immobilizing group, by including a mordant in the same layer with said image dye or by other means that would prevent the dye from diffusing from the photosensitive element.

Though the developed silver present in the photosensitive element after image formation and any remaining silver halide may be removed in a conventional manner, for example, by a bleach-fix bath, it is preferred to bleach the developed silver and to complex residual silver halide in situ. In a particularly preferred embodiment, the silver halide emulsion employed is one which upon development contains low covering power silver in the developed areas whereby the need for beaching is obviated. In these embodiments, it will be appreciated that the silver halide developing agents, the silver halide solvents and other reagents employed should be substantially non-staining.

Rather than forming monochromatic and multicolor images non-diffusible from the photosensitive element, it will be appreciated that the image dyes provided by the colorless parent compounds may be diffusible to form the color image on a single common image-receiving layer. In this embodiment, the subequent formation of a color transfer image preferably employs a differential in diffusibility between the colorless parent compound and the liberated dye. This differential in diffusibility may be achieved in a known manner by the appropriate selection of an immobilizing group(s), such as a long chain alkyl or alkoxy group and/or solubilizing group(s), such as, hydroxy, carboxy or sulfo groups.

In the latter embodiment, where the image dyes released are diffusible, the photosensitive layer and the image-receiving layer may be in separate elements which are brought together during processing and thereafter retained together as the final print or separated following image formation, or the photosensitive and image-receiving layers may be in the same element. For example, the image-receiving layer may be coated on a support and the photosensitive layer coated on the surface of the image-receiving layer. The processing composition may be applied to the combined negative-positive element using a spreader sheet to facilitate spreading the liquid composition in a uniform layer adjacent the surface of the photosensitive layer. The image-receiving layer carrying the color image may be separated from the overlying photosensitive layer(s) e.g., with the aid of a stripping layer, or the color image may be viewed as a reflection print by employing a light-reflecting layer between the photosensitive and image-receiving layers.

Illustrative of still other film units are those where the negative and positive components together comprise a unitary structure and are laminated and/or otherwise physically retained together at least prior to image formation. Generally, such film units comprise a plurality of layers including a negative component comprising at least one light-sensitive layer, e.g., a silver halide layer and a positive component comprising an image-receiving layer which components are laminated together or otherwise secured together in physical juxtaposition as a single structure.

Included among such structures are those adapted for forming a transfer image viewable without separation, i.e., wherein the positive component containing the transfer image need not be separated from the negative component for viewing purposes. In addition to the aforementioned layers, such film units include means for providing a reflecting layer between the image-receiving and negative component in order to mask effectively the silver image or images formed as a function of development of the silver halide layer or layers and also to provide a background for viewing the transfer image in the receiving component, without separation, by reflected light. This reflecting layer may comprise a preformed layer of a reflecting agent include in the film unit or the reflecting agent may be provided subsequent to photoexposure, e.g., by including the reflecting agent in the processing composition.

The aforementioned layers are preferably carried on a support and preferably are employed with another support positioned on the opposed surface of the layers carried by the first support so that the layers are sandwiched or confined between the support members, at least one of which is transparent to permit viewing of the final image. Such film units usually are employed in conjuction with means, such as, a rupturable container containing the requisite processing composition and adapted upon application of pressure of applying its contents to develop the exposed film unit. Film units of this type are now well known and are described, for example, in US—A—3,415,644, 3,415,645, 3,415,646, 3,594,164 and 3,594,165.

The processing composition employed comprises an aqueous solution and usually, an aqueous alkaline solution of a silver halide developing agent and a silver halide solvent. The named ingredients may be present initially in the aqueous medium or may be present initially in the photographic film unit, for example, in the emulsion and/or image-receiving and/or spacer layers as heretofore suggested in the art. When such ingredients are present initially in the film unit, the processing composition is formed by contacting the product with a suitable aqueous medium to form a solution of these ingredients.

The alkali employed may be any of the alkalkine materials heretofore employed, such as sodium or potassium hydroxide and like the developing agent and the solvent may be initially in a layer or layers of the film unit.

The silver halide solvent also may be any of the heretofore known materials, such as sodium or potassium thiosulfate, sodium thiocyanate or uracil; also the thioether-substituted uracils, pseudo-uracils and other compounds disclosed and claimed in US—A—4,126,459; the 1,3-disulfonylalkanes and cycloalkanes of US—A—3,769,014 and 3,958,992, respectively; or the alkanes containing an intralinear sulfonyl group and, e.g., an intralinear N-tosylsulfimido or N-tosylsulfoximido group as disclosed and claimed in US—A—43,107,176.

Also, a sivler halide solvent precursor may be used such as those disclosed in U.S. Patent No. 3,698,898 and as disclosed and claimed in US—A—4,382,119.

Examples of silver halide developing agents that may be employed are hydroquinone and substituted hydroquinones, such as tertiary butyl hydroquinone, 2,5-dimethyl hydroquinone, methoxyhydroquinone, ethoxyhydroquinone, 4'-methylphenylhydroquinone; pyrogallol and catechols, such as catechol, 4-phenyl catechol and tertiary butyl catechol; aminophenols, such as 2,4,6-diamino-orthocresol; 1,4-diaminobenzenes, such as p-phenylenediamine, 1,2,4-triaminobenzene and 4-amino-2-methyl-N,N-diethylaniline; ascorbic acid and its derivatives, such as ascorbic acid, isoascorbic acid and 5,6-isopropylidene ascorbic acid and other enediols, such as, tetramethyl reductic acid; hydroxylamines, such as N,N-di-(2-ethoxyethyl)hydroxylamine, N,N-di-(2-methoxyethyl)hydroxylamine and N,N-di-(2-methoxyethoxyethyl)-hydroxylamine; and heterocyclic compounds, such as, 1-phenyl-3-pyrazolidone and 4-methyl-4-hydroxymethyl-1-phenyl-3-pyrazolidone.

Usually, though not necessarily, the processing composition includes a viscosity-increasing reagent such as a cellulosic polymer, e.g., sodium carboxymethyl cellulose, hydroxyethyl cellulose, carboxymethyl hydroxyethyl cellulose; an oxime polymer, e.g., polydiacetone acrylamide oxime; or other high molecular weight polymers.

In addition to the aforementioned ingredients, the processing composition also may contain antifoggants, preservatives and other materials as conventionally used in the art.

The processing composition may be applied to the photosensitive element, for example, by coating, dipping, spraying or by the use of a rupturable container or pod such as disclosed in US—A—2,543,181, the container being positioned in the film unit so as to be capable upon rupturing of spreading its contents in a substantially uniform layer.

The photosensitive element may be any of these conventionally employed and generally comprises a silver halide emulsion carried on a base, for example, glass, paper or plastic film, such as cellulose triacetate film, polyethylene terephthalate film, polystyrene film and polyolefin films, e.g., polyethylene and polypropylene films. The silver halide may be a silver chloride, iodide, bromide, iodobromide, chlorobromide. The binder for the halide, though usually gelatin, may be a suitable polymer such as polvinyl alcohol, polyvinyl pyrrolidone and their copolymers.

Depending upon the particular photographic system, a mordant for the dye image may be used in association with the photosensitive layers as discussed above, or a separate image-receiving element may be employed. The image-receiving layer, i.e., dyeable stratum may comprise any of the materials known in the art, such as polyvinyl alcohol, gelatin, preferably containing a mordant for the transferred image dye(s). The dyeable stratum can be in the same element as the photosensitive layer or it may be in a separate element as appropriate for a given photographic process.

In diffusion transfer processes employing an aqueous alkaline processes composition, it is well known to employ an acid-reacting reagent in a layer of the film unit to lower the environmental pH following substantial dye transfer in order to increase the image stability. For example, the previously mentioned US—A—3,415,644 discloses systems wherein the desired pH reduction may be effected by providing an acid-reacting layer adjacent the dyeable stratum. These layers may comprise polymers which contain acid groups, e.g., carboxylic acid and sulfonic acid groups, which are capable of forming salts with alkali metals or with organic bases; or potentially acid-yielding groups such as anhydrides or lactones. Preferably the acid polymer contains free carboxyl groups. Althernatively, the acid-reacting reagent may be in a layer adjacent to the silver halide most distant from the image-receiving layer. Another system for providing an acid-reacting reagent is disclosed in US—A—3,576,625.

An inert interlayer or spacer layer may be disposed between the polymeric acid layer and the dyeable stratum in order to control or "time" the pH reduction so that it is not premature and interferes with the development process. Suitable spacer or "timing" layers for this purpose are described with particularity in US—A—3,362,819; 3,419,389; 3,421,893; 3,455,686; and 3,575,701.

The acid-reacting layer and associated spacer layer are usually contained in the image-receiving element in systems wherein the dyeable stratum and photosensitive strata are contained on separate supports, e.g., between the support for the receiving element and the dyeable stratum. In integral film units, these layers may be associated with the dyeable stratum, e.g., on the side of the dyeable stratum opposed from the photosensitive element or, if desired, they may be associated with the photosensitive strata, as is disclosed, for example, in US—A—3,362,821 and 3,573,043. In film units such as those described in the aforementioned US—A—3,594,164 and 3,594,165, they also may be contained on the spreader sheet employed to facilitate application of the processing composition.

In addition to the aforementioned layers, the film units may contain additional layers as commonly used in the art, such as a layer of antihalation dye, and/or a layer of filter dye arranged between differentially color-sensitive emulsion layers. Depending upon the particular photographic system, it may be desirable to use antihalation and filter dyes which become decolorized during photographic processing.

The following examples are given to illustrate the formation of a color image in accordance with the present invention and are not intended to be limiting.

## Example 9

A photosensitive element was prepared by coating a transparent gel subcoated polyester film base with the following layers:

1. a layer of the hydrochloride salt of the compound of Formula 1 coated at a coverage of 969 mg/m$^2$ in gelatin, said hydrochloride salt having the formula

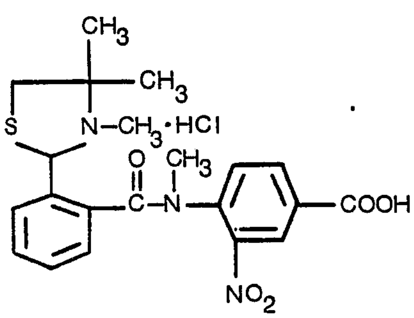

;

and

2. a gelatino silver iodobromide emulsion coated at a coverage of 533—646 mg/m$^2$ silver.

The photosensitive element was exposed imagewise and processed against an image-receiving element comprising a layer of dyeable film-forming material coated on a baryta paper base at a gap of 0.076 mm using an aqueous alkaline processing composition containing the following in % by weight:

| | |
|---|---|
| Carboxymethyl cellulose | 4.2 |
| Sodium hydroxide | 2.4 |
| Sodium sulfite | 2.0 |
| Sodium thiosulfate | 0.5 |
| Tetramethylreductic acid and water to make 100% | 1.0 |

After an imbibition time of three minutes in the dark, the respective elements were separated to show a positive yellow dye image.

The solution kinetics of the hydrochloride salt of the compound of Formula 1 also were studied in aqueous alkaline solution in the presence and in the absence of soluble silver complex. It was found that this compound hydrolized with a half life ($T_{\frac{1}{2}}$) of 24 seconds in the presence of soluble silver (0.8 mol of NaOH, $10^{-3}$ mol $Ag_2S_2O_3$, $10^{-5}$ mol Ex. 1 HCl salt; 25°C) and hydrolyzed with a $T_{\frac{1}{2}} = 115$ hours in the absence of soluble silver (1.0 N aq. NaOH, $10^{-5}$ mol Ex. 1 HCl salt; 25°C).

The solution kinetics of the compound of Formula 2 also were studied. This compound was nearly colorless in solution and when treated with alkaline silver thiosulfate gave yellow azo dye. The azo dye formed with a $T_{\frac{1}{2}} = 32$ hours in the absence of soluble silver (0.5 N aq. NaOH, $5 \times 10^{-5}$ mol Ex. 2; 25°C) and with a $T_{\frac{1}{2}} = 11$ seconds in the presence of soluble silver (0.5 N aq. NaOH, $10^{-3}$ mol $Ag_2S_2O_3$, 25°C). A plot of the optical density versus time for azo dye formation gave an "S" shaped curve, typical of $A \xrightarrow{T_{\frac{1}{2}}^{a}} B \xrightarrow{T_{\frac{1}{2}}^{b}} C$ reaction, from which values of $T_{\frac{1}{2}}^{a} = 1$ to 3 seconds and $T_{\frac{1}{2}}^{b} = 10$ seconds could be estimated.

## Example 10

Two photosensitive elements were prepared by coating a gelatin subcoated polyethylene terephthalate film base with a gelatino silver iodobromide emulsion. One of said photosensitive elements was coated at a coverage of 215 mg/m² of silver, and the other of said elements was coated at a coverage of 430 mg/m² of silver.

A second element containing a compound of the present invention was prepared employing a component similar to that described in US—A—3,647,437 (column 66, lines 46 to 59) which comprised a transparent polyethylene terephthalate film base carrying the following layers.

(1) a polymeric acid layer
(2) a polymeric spacer (timing) layer
(3) a polymeric image-receiving layer.

To form said second element, the above component was coated (over the image-receiving layer) with

(a) 1076 mg/m² of poly-4-vinylpyridine containing 1076 mg/m² of the colorless image dye-providing compound of Formula 3 (or 1076 mg/m² (for a separate second element) of the colorless image dye-providing compound of Formula 4) and (b) 323 mg/m² of gelatin containing succindialdehyde hardener.

The photosensitive elements were given an exposure through a stepwedge to white light of 2 mcs and superposed with said second elements. A layer of an aqueous alkaline processing composition 0,05 mm thick was distributed between said elements by passing the film units between a pair of pressure-applying rolls in the dark. The processing composition comprised the following ingredients.

| | |
|---|---|
| Water | 100 ml |
| Potassium hydroxide | 5 g |
| Sodium sulfite | 2 g |
| 6-methylthiomethyl-2,4-dihydroxy-pyrimidine | 1.5 g |
| Tetramethyl reductic acid | 6 g |
| Hydroxyethyl cellulose | 3 g |
| Titanium dioxide | 50 g |

After applying said processing composition the film units were maintained intact to provide an integral negative-positive reflection print, and at recorded time intervals, the maximum and minimum reflection densities were measured for the positive yellow images. The densities measured at given times are set forth in Table I below.

TABLE I

| Cpd of Formula 3 215 mg/m² Ag Time | Dmax/Dmin | Cpd of Formula 3 430 mg/m² Ag Time | Dmax/Dmin | Cpd of Formula 4 430 mg/m² Ag Time | Dmax/Dmin |
|---|---|---|---|---|---|
| 10 min | 0.37/0.25 | 10 min | — | 10 min | 0.38/0.33 |
| 20 min | 0.40/0.27 | 20 min | 0.44/0.24 | — | — |
| 30 min | 0.40/0.27 | — | — | — | — |
| 40 min | 0.41/0.27 | 75 min | 0.49/0.27 | 40 min | 0.50/0.32 |
| 2.5 h | 0.47/0.28 | 3.5 h | 0.52/0.29 | 2.5 h | 0.95/0.52 |
| 18 h | 0.60/0.54 | 18 h | 0.64/0.56 | — | — |

Example 11

A photosensitive element was prepared by coating a gelatin subcoated polyethylene terephthalate film base with a gelatino silver iodobromide emulsion at a coverage of 430 mg/m$^2$ of silver.

A second element was prepared in the same manner described in Example 10 above except that the coating (a) contained 1615 mg/m$^2$ of the compound of Formula 6 in 1076 mg/m$^2$ of poly-4-vinylpyridine.

The unexposed photosensitive element was superposed with said second element and one-half of the resulting sandwich was processed with a layer (0.05 mm thick) of an aqueous 5% sodium hydroxide solution containing 3% of hydroxyethyl cellulose and the other half of the sandwich was processed with a layer (0.05 mm thick) of an aqueous 5% sodium hydroxide solution containing 1.5% of 6-methylthiomethyl-2,4-dihydroxypyrimidine silver halide solvent and 3% of hydroxyethyl cellulose. The optical transmission densities were measured at given time intervals for the two halves of the sandwich and showed the formation of dye from the colorless precursor. The results obtained are set forth in Table II below.

TABLE II

| Time (min) | Optical Transmission Density Without Solvent | With Solvent |
|---|---|---|
| 10 | 0.27 | 0.27 |
| 27 | 0.24 | 0.32 |
| 39 | 0.25 | 0.55 |
| 57 | 0.28 | 0.73 |

Example 12

A photosensitive element was prepared by coating a transparent polyethylene terephthalate film base with the following layers.

(1) a layer containing 538 mg/m$^2$ of poly-4-vinylpyridine and 1615 mg/m$^2$ of the compound of Formula 5,

(2) a layer of gelatin coated at a coverage of 1076 mg/m$^2$,

(3) a gelatino silver iodobromide emulsion layer coated at a coverage of 646 mg/m$^2$ of silver, and

(4) a layer of gelatin containing succindialdehyde.

To show the formation of dye in the presence of silver halide complex, the unexposed photosensitive element was superposed with a second element similar to that described in US—A—3,647,437 (column 66, lines 46 to 59) which comprised a transparent polyethylene terephthalate film base carrying the following layers:

(1) a polymeric acid layer

(2) a polymeric spacer (timing) layer

(3) a polymeric image-receiving layer.

The superposed elements were processed in the same manner using the same two processing compositions as in Example 11 above to show the formation of dye from the colorless precursor in the presence of silver halide complex. The optical transmission densities that were measured at given time intervals are set forth in Table III below.

TABLE III

| Time | Optical Transmission Density Without Solvent | With Solvent |
|---|---|---|
| 1 min | 0.13 | 0.14 |
| 3 min | 0.14 | 0.15 |
| 10 min | 0.18 | 0.20 |
| 1 h | 0.17 | 0.26 |
| 1 day | 0.26 | 0.36 |
| 7 day | 0.45 | 0.60 |

Example 13

A photosensitive element was prepared by coating a transparent polyethylene terephthalate film base with the following layers.

(1) A layer of gelatin containing the compound of Formula 7,

(2) a gelatino silver iodobromide emulsion layer coated at a coverage of 215 mg/$m^2$ of silver, and

(3) a layer of gelatin containing succindialdehyde.

The unexposed photosensitive element was superposed with a second element comprising a transparent polyethylene terephthalate film base coated with the following layers.

(1) as a polymeric acid layer, a mixture of 9 parts of a partial butyl ester of polyethylene/maleic anhydride copolymer and 1 part of polyvinyl butyral coated at a coverage of 26.9 g/$m^2$;

(2) a timing layer containing a 14:1 ratio of a 60—30—4—6 tetrapolymer of butylacrylate, diacetone acrylamide, styrene and methacrylic acid and polyvinyl alcohol at a coverage of 5.38 g/$m^2$; and

(3) a blend of 3 parts by weight of a 2:1 mixture, by weight, of polyvinyl alcohol and poly-4-vinylpyridine and 1 part by weight of a graft copolymer of 4-vinylpyridine and vinylbenzyltrimethylammonium-chloride grafted on hydroxyethyl cellulose in a weight ratio of 2.2/1/2.2, respectively, coated at a coverage of 3.23 g/$m^2$ to provide an image-receiving layer.

The resulting sandwich was processed by applying a layer (0.05 mm thick) of an aqueous 7% sodium hydroxide solution containing 2.5% of carboxymethyl hydroxyethyl cellulose to one-half of the sandwich and applying to the other half, a layer (0.05 mm thick) an aqueous 7% sodium hydroxide solution containing 1.5% of 6-methylthiomethyl-2,4-dihydroxypyrimidine silver halide solvent, 0.009% of 2-thiouracil and 2.5% of carboxymethyl hydroxyethyl cellulose. After 10 minutes, the optical transmission density measured for the half processed without silver halide solvent was 0.16 and the density measured for the half processed with silver halide solvent was 0.24.

An identical photosensitive element was processed in the same manner described above except that a transparent polyethylene terephthalate sheet was employed as the second element. The optical transmission densities measured for the no silver solvent-silver solvent portions of the sandwich like those above, also showed formation of dye from the colorless precursor in the presence of silver halide complex and were 0.35 and 0.73, respectively.

Example 14

A photosensitive element was prepared by coating a transparent polyethylene terephthalate film base with the following layers.

(1) a layer containing 592 mg/$m^2$ of cellulose acetate hydrogen phthalate and 1.184 g/$m^2$ the compound of Formula 8,

(2) a gelatino silver iodobromide emulsion layer coated at a coverage of 269 mg/$m^2$ of silver and containing 323 mg/$m^2$ of 4'-methylphenylhydroquinone, and

(3) a layer of gelatin coated at a coverage of 323 mg/$m^2$.

A second element was prepared by coating a transparent polyethylene terephthalate film base with the following layers.

(1) as a polymeric acid layer, a mixture of 9 parts of a partial butyl ester of polyethylene/maleic anhydride copolymer and 1 part of polyvinyl butyral coated at a coverage of 26.9 g/$m^2$;

(2) a timing layer containing a 14:1 ratio of a 60—30—4—6 tetrapolymer of butylacrylate, a diacetone acrylamide, styrene and methacrylic acid and polyvinyl alcohol at a coverage of 5.38 g/$m^2$;

(3) a blend of 3 parts by weight of a 2:1 mixture, by weight, of polyvinyl alcohol and poly-4-vinylpyridine and 1 part by weight of a graft copolymer of 4-vinylpyridine and vinylbenzyltrimethyl-ammoniumchloride grafted on hydroxyethyl cellulose in a weight ratio of 2.2/1/2.2, respectively, coated at a coverage of 3.23 g/$m^2$ to provide an image-receiving layer.

One half of the photosensitive element was exposed to white light, the other half being left unexposed. The photosensitive element was superposed with said second element and the sandwich was processed by applying a layer of processing composition (0.25 mm thick) containing the following ingredients.

| | |
|---|---|
| Water | 100 ml |
| Potassium hydroxide | 14 g |
| 6-butylthiomethyl-2,4-dihydroxypyrimidine | 3 g |
| Carboxymethyl hydroxyethyl cellulose | 2.5 g |

After 5 minutes in the dark, the optical transmission densities measured for the exposed and unexposed portions of the film unit were 0.27 and 0.57, respectively.

It will be appreciated that the photographic systems of the present invention for providing a color image may be used with film structures other than those illustrated.

**Claims**

1. Photographic product comprising a support layer carrying, on one side thereof, a light-sensitive silver halide layer and a colourless precursor M—Z of a preformed image dye, having a moiety and a radical Z, said moiety M comprising a 1,3-sulfur-nitrogen group A possessing a carbon atom in the two-position, intermediate said sulfur and nitrogen atoms, and undergoing cleavage in the presence of silver ions and/or soluble silver complex and said radical Z being the radical of the colorless precursor of a preformed image dye, characterized in that said moiety M is substituted on said radical Z such that said colorless precursor M—Z is maintained in its colorless form until said 1,3-sulfur-nitrogen group A undergoes cleavage.

2. Photographic product according to claim 1 characterized in that said colorless precursor M—Z is non-diffusible in aqueous alkaline solution.

3. Photographic product according to claims 1 or 2 characterized in that said image dye provided by said colorless precursor M—Z is diffusible in aqueous alkaline solution.

4. Photographic product according to any preceding claim characterized in that it additionally includes means for applying an aqueous alkaline developer liquid containing a silver halide developing agent and a silver halide solvent.

5. Photographic product according to claim 4 characterized in that at least one of said developing agent and said solvent is contained initially in a layer of said photographc product.

6. Photographic product according to any preceding claim characterized in that said colorless precursor M—Z is positioned in said silver halide layer.

7. Photographic product according to any preceding claim characterized in that said silver halide is negative working.

8. Photographic product according to any preceding claim characterized in that it includes an image-receiving layer adapted for providing a color transfer image by diffusion of dye thereto.

9. Photographic product according to claim 8 characterized in that it includes a light-reflecting layer between said silver halide and image-receiving layers, whereby a color transfer image formed in said image receiving layer may be viewed by reflection, said light-reflecting layer being effective to mask the developed silver halide layer from one viewing said color image.

10. Photographic product according to any preceding claim characterized in that it includes an acid-reacting layer.

11. Photographic product according to claim 1 characterized in that said image dye is non-diffusible from the layer in which it is positioned during photoexposure.

12. Photographic product according to any preceding claim characterized in that said 1,3-sulfur-nitrogen group A of said moiety M of said colorless precursor M—Z possesses an amide substituent L on the carbon atom in the 2-position which upon cleavage of said 1,3-sulfur-nitrogen group A undergoes an intramolecularly accelerated cleavage reaction.

13. Photographic product according to claim 12 characterized in that said substituent L in the 2-position of said 1,3-sulfur-nitrogen group A is a carboxamido substituent.

14. Photographic product according to claim 13 characterized in that said 1,3-sulfur-nitrogen group A is a thiazolidin-2-yl group.

15. Photographic product according to claim 12 characterized in that said colorless precursor M—Z is of the formula:

$$A—L—Z^1$$

wherein A and L are defined as in claim 12, and $Z^1$ is a preformed image dye rest which when taken with L is the colorless precursor radical Z of a preformed image dye.

16. Photographic product according to claim 15 characterized in that said colorless precursor M—Z is of the formula

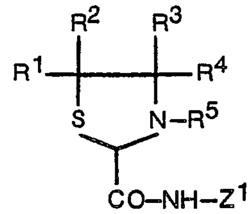

wherein $R^1$, $R^2$ and $R^3$ each are selected from hydrogen, alkyl, aryl, aralkyl and alkaryl; $R^4$ is selected from hydrogen, carboxy, N,N-dialkylcarboxamido, alkyl, aryl, aralkyl and alkaryl; $R^5$ is selected from alkyl, aryl, alkaryl and aralkyl; and $Z^1$ is a preformed image dye rest which when taken with said —CO—NH— is the colorless precursor radical Z of a preformed image dye.

17. Photographic product according to claim 15 characterized in that said colorless precursor M—Z is of the formula:

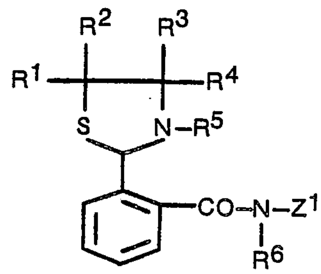

wherein $R^1$, $R^2$ and $R^3$ each are selected from hydrogen, alkyl, aryl, aralkyl and alkaryl; $R^4$ is selected from hydrogen, carboxy, N,N-dialkylcarboxamido, alkyl, aryl, aralkyl and alkaryl; $R^5$ is selected from alkyl, aryl, alkaryl and aralkyl; $R^6$ is alkyl or aryl; and $Z^1$ is a preformed image dye rest which when taken with said

$$-CO-\underset{\displaystyle R^6}{\underset{|}{N}}-$$

is the colorless precursor radical Z of a preformed image dye.

18. Photographic product according to any of claims 1 to 11 characterized in that said 1,3-sulfur-nitrogen group A of said moiety M of said colorless precursor M—Z is a thiazolidinyl group T possessing a substituent $L^1$ on the carbon atom in the 2-position which upon cleavage of said thiazolidinyl group, undergoes a beta-elimination reaction.

19. Photographic product according to claim 18 characterized in that said beta-elimination reaction is followed by an intramolecularly accelerated nucleophilic displacement reaction.

20. Photographic product according to claim 19 characterized in that said beta-elimination reaction effects cleavage of an amide group.

21. Photographic product according to claim 20 characterized in that said amide group is a carboxamido group.

22. Photographic product according to claim 18 characterized in that said colorless precursor M—Z is of the formula

$$T^1—L^1—Y—Z^1$$

wherein $T^1$ is a thiazolidin-2′-yl group capable of undergoing cleavage in the presence of silver ions and/or soluble silver complex; $L^1$ is defined as in claim 18; Y is a leaving group released by said beta-elimination reaction; and $Z^1$ is a preformed image dye rest which when taken with Y is the colorless precursor radical Z of said preformed image dye, said $T^1$—$L^1$— being substituted on said precursor radical Z such that the colorless precursor M—Z is maintained in its colorless form until said thiazolidin-2′-yl group undergoes said cleavage.

23. Photographic product according to claim 22 characterized in that said colorless precursor M—Z is of the formula

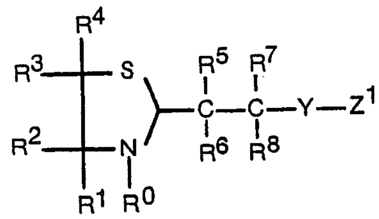

wherein $R^0$ is selected from alkyl, aryl, aralkyl and alkaryl; $R^1$ is selected from hydrogen, carboxy, N,N-dialkylcarboxamido, alkyl, aryl, aralkyl and alkaryl; $R^2$, $R^3$ and $R^4$ each are selected from hydrogen, alkyl, aryl, aralkyl and alkaryl; $R^5$ is selected from hydrogen and a group that can be removed upon cleavage of said thiazolidinyl group to leave an electron pair; $R^6$, $R^7$ and $R^8$ each are selected from hydrogen, alkyl, aryl, aralkyl and alkaryl; and Y is defined as in claim 22; and $Z^1$ is a preformed image dye rest which when taken with Y is the colorless precursor radical Z of a preformed image dye.

24. Photographic product according to claim 19 characterized in that said colorless precursor M—Z is of the formula:

$$T^1—L^1—Y^1—Z^1$$

wherein $T^1$ is a thiazolidin-2′-yl group capable of undergoing cleavage in the presence of silver ions and/or soluble silver complex; $L^1$ is defined as in claim 18; $Y^1$ is a moiety capable of undergoing an intramolecularly accelerated nucleophilic displacement reaction following said beta-elimination reaction; and $Z^1$ is a preformed image dye rest which when taken with $Y^1$ is the colorless precursor radical Z of a preformed image dye, said $T^1—L^1—$ being substituted on said colorless precursor radical such that the colorless precursor M—Z is maintained in its colorless form until said thiazolidin-2′-yl group undergoes said cleavage.

25. Photographic process comprises the steps of photoexposing a photographic product including a silver halide emulsion having associated therewith a colorless precursor of a preformed image dye; and developing said exposed silver halide emulsion to form, as a function of development, a color image in dye from said colorless precursor; characterized in that said photographic product is a product according to any preceding claim.

26. Colorless dye-providing compound having the formula

$$M—Z$$

wherein M is a moiety comprising a 1,3-sulfur-nitrogen group A possessing a carbon atom in the 2-position intermediate said sulfur and nitrogen atoms, and undergoing cleavage in the presence of silver ions and/or soluble silver complex, and Z is the radical of a colorless precursor of a preformed image dye, characterized in that said M moiety is substituted on said radical Z such that said colorless precursor is maintained in its colorless form until said 1,3-sulfur-nitrogen group A undergoes cleavage.

27. Colorless dye-providing compound according to claim 26 characterized in that said compound M—Z is of the formula

$$A—L—Z^1$$

wherein A is defined as in claim 26; L is an amide moiety capable of undergoing an intramolecularly accelerated cleavage reaction following the cleavage of said 1,3-sulfur-nitrogen group; and $Z^1$ is a preformed image dye rest which when taken with L is the colorless precursor radical Z of a preformed image dye.

28. Colorless dye-providing compound according to claim 27 characterized in that said compound is of the formula:

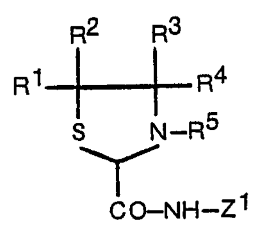

wherein $R^1$, $R^2$ and $R^3$ each are selected from hydrogen, alkyl, aryl, aralkyl and alkaryl; $R^4$ is selected from hydrogen, carboxy, N,N-dialkylcarboxamido, alkyl, aryl, aralkyl and alkaryl; $R^5$ is selected from alkyl, aryl,

alkaryl and aralkyl; and $Z^1$ is a preformed image dye rest which when taken with said —CO—NH— is the colorless precursor radical Z of a preformed image dye.

29. Colorless dye-providing compound according to claim 27 characterized in that said compound M—Z is of the formula:

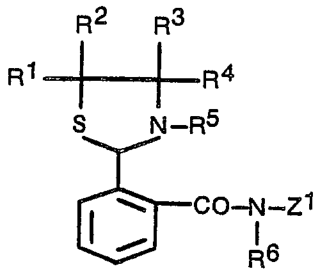

wherein $R^1$, $R^2$ and $R^3$ each are selected from hydrogen, alkyl, aryl, aralkyl and alkaryl; $R^4$ is selected from hydrogen, carboxy, N,N-dialkylcarboxamido, alkyl, aryl, aralkyl and alkaryl; $R^5$ is selected from alkyl, aryl, alkaryl and aralkyl; $R^6$ is alkyl or aryl; and $Z^1$ is a preformed image dye rest which when taken with said

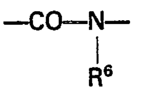

is the colorless precursor radical Z of a preformed image dye.

30. Colorless dye-providing compound according to claim 26 characterized in that said compound M—Z is of the formula:

$$T^1—L^1—Y—Z^1$$

wherein $T^1$ is a thiazolidin-2′-yl group capable of undergoing cleavage in the presence of silver ions and/or soluble silver complex; $L^1$ is a moiety capable of undergoing a beta-elimination reaction following the cleavage of said thiazolidin-2′-yl group; Y is a leaving group released by said beta-elimination reaction; and $Z^1$ is a preformed image dye rest which when taken with Y is the colorless precursor radical Z of said preformed image dye, said $T^1$—$L^1$ being substituted on said precursor radical Z such that the colorless precursor is maintained in its colorless form until said thiazolidin-2′-yl group undergoes said cleavage.

31. Colorless dye-providing compound according to claim 30 characterized in that said compound is of the formula

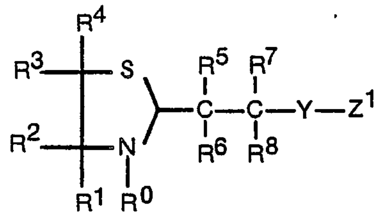

wherein $R^0$, is selected from alkyl, aryl, aralkyl and alkaryl; $R^1$ is selected from hydrogen, carboxy, N,N-dialkylcarboxamido, alkyl, aryl, aralkyl and alkaryl; $R^2$, $R^3$ and $R^4$ each are selected from hydrogen, alkyl, aryl, aralkyl and alkaryl; $R^5$ is selected from hydrogen and a group that can be removed upon cleavage of said thiazolidinyl group to leave an electron pair; $R^6$, $R^7$ and $R^8$ each are selected from hydrogen, alkyl, aryl, aralkyl and alkaryl; and Y and $Z^1$ are defined as in claim 30.

32. Colorless dye-providing compound according to claim 26 characterized in that said compound M—Z is of the formula:

$$T^1—L^1—Y^1—Z^1$$

wherein $T^1$ and $L^1$ are defined as in claim 30; $Y^1$ is a moiety capable of undergoing an intramolecularly accelerated nucleophilic displacement reaction following said beta-elimination reaction; and $Z^1$ is a preformed image dye rest which when taken with $Y^1$ is the colorless precursor radical Z of a preformed image dye, said $T^1$—$L^1$ being substituted on said colorless precursor radical such that the colorless precursor M—Z is maintained in its colorless form until said thiazolidin-2′-yl group undergoes said cleavage.

33. Colorless dye-providing compound according to claim 32, characterized in that said compound is of the formula

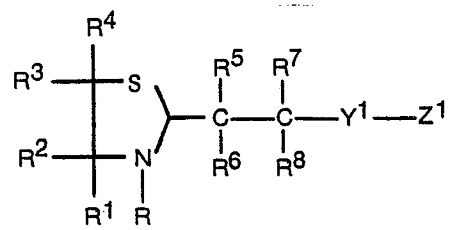

wherein R is selected from alkyl, aryl, aralkyl and alkaryl; $R^1$ is selected from hydrogen, carboxy, N,N-dialkylcarboxamido, alkyl, aryl, aralkyl and alkaryl; $R^2$, $R^3$ and $R^4$ each are selected from hydrogen, alkyl, aryl, aralkyl and alkaryl; $R^5$ is selected from hydrogen and a group that can be removed upon cleavage of said thiazolidinyl group to leave an electron pair; $R^6$, $R^7$ and $R^8$ each are selected from hydrogen, alkyl, aryl, aralkyl and alkaryl; $Y^1$ and $Z^1$ are defined as in claim 32.

**Patentansprüche**

1. Photographisches Produkt, enthaltend eine Trägerschicht, die auf einer Seite eine lichtempfindliche Silberhalogenidschicht und eine farblose Vorstufe M—Z eines vorgeformten Bildfarbstoffs trägt, der eine Gruppe M und einen Rest Z trägt, wobei die Gruppe M eine 1,3-Schwefel-Stickstoff-Gruppe A enthält, die ein Kohlenstoffatom in 2-Stellung zwischen dem Schwefel- und Stickstoffatom trägt und die in Gegenwart von Silberionen und/oder einem löslichen Silberkomplex einer Spaltung unterliegt, wobei der Rest Z der Rest der farblosen Vorstufe eines vorgeformten Bildfarbstoffs ist, dadurch gekennzeichnet, daß die Gruppe M so am Rest Z substituiert ist, daß die farblose Vorstufe M—Z in ihrer farblosen Form gehalten wird, bis die 1,3-Schwefel-Stickstoff-Gruppe A gespalten wird.

2. Photographisches Produkt nach Anspruch 1, dadurch gekennzeichnet, daß die farblose Vorstufe M—Z in wäßrig-alkalischer Lösung nicht diffundierbar ist.

3. Photographisches Produkt nach Anspruch 1 oder 2, dadurch gekennzeichnet, daß der durch die farblose Vorstufe M—Z bereitgestellte Bildfarbstoff in wäßrig-alkalischer Lösung diffundierbar ist.

4. Photographisches Produkt nach einem vorhergehenden Anspruch, dadurch gekennzeichnet, daß es zusätzlich Mittel zum Aufbringen einer wäßrig-alkalischen Entwicklerflüssigkeit umfaßt, die einen Silberhalogenid-Entwickler und ein Silberhalogenid-Lösungsmittel enthält.

5. Photographisches Produkt nach Anspruch 4, dadurch gekennzeichnet, daß zumindest der Entwickler bzw. das Lösungsmittel ursprünglich in einer Schicht des photographischen Produktes enthalten sind.

6. Photographisches Produkt nach einem vorhergehenden Anspruch, dadurch gekennzeichnet, daß die farblose Vorstufe M—Z in der Silberhalogenidschicht angeordnet ist.

7. Photographisches Produkt nach einem vorhergehenden Anspruch, dadurch gekennzeichnet, daß das Silberhalogenid negativ arbeitend ist.

8. Photographisches Produkt nach einem vorhergehenden Anspruch, dadurch gekennzeichnet, daß es eine Bildempfangsschicht enthält, die zur Erzeugung eines farbigen Übertragungsbildes durch Farbstoffdiffusion eingerichtet ist.

9. Photographisches Produkt nach Anspruch 8, dadurch gekennzeichnet, daß es eine das Licht reflektierende Schicht zwischen der Silberhalogenidschicht und der Bildempfangsschicht enthält, wodurch ein in der Bildempfangsschicht erzeugtes farbiges Übertragungsbildung durch Reflexion betrachtbar ist, wobei die das Licht reflektierende Schicht dahingehend wirkt, daß sie die entwickelte Silberhalogenidschicht gegenüber einem Betrachter des Farbbildes abdeckt.

10. Photographisches Produkt nach einem vorhergehenden Anspruch, dadurch gekennzeichnet, daß es eine sauer reagierende Schicht enthält.

11. Photographisches Produkt nach Anspruch 1, dadurch gekennzeichnet, daß der Bildfarbstoff aus der Schicht, in welcher er sich während der Belichtung befindet, nicht diffundierbar ist.

12. Photographisches Produkt nach einem vorhergehenden Anspruch, dadurch gekennzeichnet, daß die 1,3-Schwefel-Stickstoff-Gruppe A der Gruppe M der farblosen Vorstufe M—Z einen Amidsubstituenten L am Kohlenstoffatom in der 2-Stellung besitzt, der nach der Spaltung der 1,3-Schwefel-Stickstoff-Gruppe A einer intramolekular beschleunigten Spaltreaktion unterliegt.

13. Photographisches Produkt nach Anspruch 12, dadurch gekennzeichnet, daß der Substituent L in der 2-Stellung der 1,3-Schwefel-Stickstoff-Gruppe A ein Carboxamido-Substituent ist.

14. Photographisches Produkt nach Anspruch 13, dadurch gekennzeichnet, daß die 1,3-Schwefel-Stickstoff-Gruppe A eine Thiazolidin-2-yl-Gruppe ist.

15. Photographisches Produkt nach Anspruch 12, dadurch gekennzeichnet, daß die farblose Vorstufe M—Z die Formel

$$A—L—Z^1$$

hat, worin A und L wie in Anspruch 12 definiert sind und $Z^1$ ein vorgeformter Bildfarbstoffrest ist, der zusammen mit L den Rest Z der farblosen Vorstufe eines vorgeformten Bildfarbstoffs darstellt.

16. Photographisches Produkt nach Anspruch 15, dadurch gekennzeichnet, daß die farblose Vorstufe M—Z die Formel

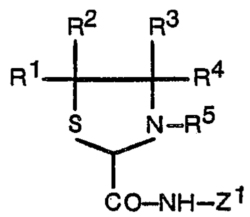

hat, worin bedeuten: $R^1$, $R^2$ und $R^3$ sind jeweils aus Wasserstoff, Alkyl, Aryl, Aralkyl und Alkaryl ausgewählt; $R^4$ ist aus Wasserstoff, Carboxy, N,N-Dialkylcarboxamido, Alkyl, Aryl, Aralkyl und Alkaryl, ausgewählt; $R^5$ ist aus Alkyl, Aryl, Alkaryl und Aralkyl ausgewählt; und Z ist der Rest eines vorgeformten Bildfarbstoffs, der zusammen mit der Gruppe —CO—NH— den Rest Z der farblosen Vorstufe eines vorgeformten Bildfarbstoffs darstellt.

17. Photographisches Produkt nach Anspruch 15, dadurch gekennzeichnet, daß die farblose Vorstufe M—Z die Formel

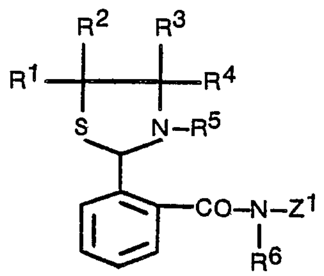

hat, worin bedeuten: $R^1$, $R^2$ und $R^3$ sind jeweils aus Wasserstoff, Alkyl, Aryl, Aralkyl und Alkaryl ausgewählt; $R^4$ ist aus Wasserstoff, Carboxy, N,N-Dialkylcarboxamido, Alkyl, Aryl, Aralkyl und Alkaryl ausgewählt; $R^5$ ist aus Alkyl, Aryl, Alkaryl und Aralkyl ausgewählt; $R^6$ ist Alkyl oder Aryl; und $Z^1$ ist der Rest eines vorgeformten Bildfarbstoffs, der zusammen mit der Gruppe

—CO—N—
|
$R^6$

den Rest Z der farblosen Vorstufe eines vorgeformten Bildfarbstoffs darstellt.

18. Photographisches Produkt nach einem der Ansprüche 1 bis 11, dadurch gekennzeichnet, daß die 1,3-Schwefel-Stickstoff-Gruppe A der Gruppe M der farblosen Vorstufe M—Z eine Thiazolidinylgruppe T mit einem Substituenten $L^1$ am Kohlenstoffatom in der 2-Stellung ist, der bei der Spaltung der Thiazolidinylgruppe einer β-Eliminierungsreaktion unterliegt.

19. Photographisches Produkt nach Anspruch 18, dadurch gekennzeichnet, daß sich an die β-Eliminierungsreaktion eine intramolekular beschleunigte nukleophile Verdrängungsreaktion anschließt.

20. Photographisches Produkt nach Anspruch 19, dadurch gekennzeichnet, daß die β-Eliminierungsreaktion die Spaltung einer Amidgruppe bewirkt.

21. Photographisches Produkt nach Anspruch 20, dadurch gekennzeichnet, daß die Amidgruppe eine Carboxamidogruppe ist.

22. Photographisches Produkt nach Anspruch 18, dadurch gekennzeichnet, daß die farblose Vorstufe M—Z die Formel

$$T^1—L^1—Y—Z^1$$

hat, worin bedeuten; $T^1$ ist eine Thiazolidin-2'-yl-Gruppe, die in Gegenwart von Silberionen und/oder einem löslichen Silberkomplex einer Spaltung unterliegt; $L^1$ ist wie in Anspruch 18 definiert; Y ist eine austretende Gruppe, die durch die β-Eliminierungsreaktion freigesetzt wird; und $Z^1$ ist der Rest eines vorgeformten Bildfarbstoffs, der zusammen mit Y den Rest Z der farblosen Vorstufe des vorgeformten Bildfarbstoffs darstellt, wobei $T^1—L^1—$ an dem Rest Z der Vorstufe substituiert ist, so daß die farblose Vorstufe M—Z in ihrer farblosen Form gehalten wird, bis die Thiazolidin-2'-yl-Gruppe der Spaltung unterliegt.

23. Photographisches Produkt nach Anspruch 22, dadurch gekennzeichnet, daß die farblose Vorstufe M—Z die Formel

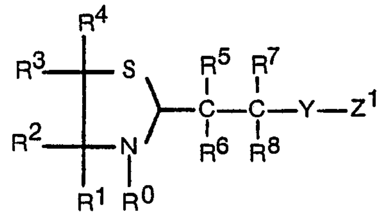

hat, worin bedeuten: $R^0$ ist aus Alkyl, Aryl, Aralkyl und Alkaryl ausgewählt; $R^1$ ist aus Wasserstoff, Carboxy, N,N-Dialkylcarboxamido, Alkyl, Aryl, Aralkyl und Alkaryl ausgewählt; $R^2$, $R^3$ und $R^4$ sind jeweils aus Wasserstoff, Alkyl, Aryl, Aralkyl und Alkaryl ausgewählt; $R^5$ ist aus Wasserstoff und einer Gruppe ausgewählt, die bei der Spaltung der Thiazolidinylgruppe unter Zurücklassung eines Elektronenpaars entfernt werden kann; $R^6$, $R^7$ und $R^8$ sind jeweils aus Wasserstoff, Alkyl, Aryl, Aralkyl und Alkaryl ausgewählt; Y ist wie in Anspruch 22 definiert; und $Z^1$ ist der Rest eines vorgeformten Bildfarbstoffs, der zusammen mit Y den Rest Z der farblosen Vorstufe eines vorgeformten Bildfarbstoffs darstellt.

24. Photographisches Produkt nach Anspruch 19, dadurch gekennzeichnet, daß die farblose Vorstufe M—Z die Formel

$$T^1—L^1—Y—Z^1$$

hat, worin bedeuten: $T^1$ ist eine Thiazolidin-2'-yl-Gruppe, die in Gegenwart von Silberionen und/oder einem löslichen Silberkomplex einer Spaltung unterliegt; $L^1$ ist wie in Anspruch 18 definiert; $Y^1$ ist eine Gruppe, die in der Lage ist, eine intramolekular beschleunigte nukleophile Verdrängungsreaktion nach der β-Eliminierungsreaktion auszuführen; und $Z^1$ ist der Rest eines vorgeformten Bildfarbstoffs, der zusammen mit $Y^1$ den Rest Z einer farblosen Vorstufe eines vorgeformten Bildfarbstoffs darstellt, wobei $T^1—L^1—$ an dem Rest der farblosen Vorstufe substituiert ist, so daß die farblose Vorstufe M—Z in ihrer farblosen Form gehalten wird, bis die Thiazolidin-2'-yl-Gruppe der Spaltung unterliegt.

25. Photographisches Verfahren, das folgende Schritte umfaßt: Belichtung eines photographischen Produkts, enthaltend eine Silberhalogenidemulsion, der eine farblose Vorstufe eines vorgeformten Bildfarbstoffs zugeordnet ist; und Entwicklung der belichteten Silberhalogenidemulsion, um als Funktion der Entwicklung ein farbiges Farbstoffbild aus der farblosen Vorstufe zu bilden; dadurch gekennzeichnet, daß das photographische Produkt ein Produkt nach einem der vorhergehenden Ansprüche darstellt.

26. Farblose, einen Farbstoff bildende Verbindung der Formel

M—Z,

worin bedeuten: M ist eine Gruppe, die eine 1,3-Schwefel-Stickstoff-Gruppe A mit einem Kohlenstoffatom in der 2-Stellung zwischen dem Schwefel- und dem Stickstoffatom enthält und die in Gegenwart von Silberionen und/oder einem löslichen Silberkomplex einer Spaltung unterliegt; und Z ist der Rest einer farblosen Vorstufe eines vorgeformten Bildfarbstoffs, dadurch gekennzeichnet, daß die Gruppe M so am Rest Z substituiert ist, daß die farblose Vorstufe in ihrer farblosen Form gehalten wird, bis die 1,3-Schwefel-Stickstoff-Gruppe A der Spaltung unterliegt.

27. Farblose, einen Farbstoff bildende Verbindung nach Anspruch 26, dadurch gekennzeichnet, daß die Verbindung M—Z die Formel

$$A—L—Z^1$$

hat, worin bedeuten: A ist wie in Anspruch 26 definiert; L ist eine Amidgruppe, die in der Lage ist, eine intramolekular beschleunigte Spaltreaktion dach der Spaltung der 1,3-Schwefel-Stickstoff-Gruppe enzugehen; und $Z^1$ ist der Rest eines vorgeformten Bildfarbstoffs, der zusammen mit L den Rest Z der farblosen Vorstufe eines vorgeformten Bildfarbstoffs darstellt.

28. Farblose, einen Farbstoff bildende Verbindung nach Anspruch 27, dadurch gekennzeichnet, daß die Verbindung die Formel

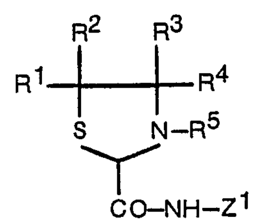

hat, worin bedeuten: $R^1$, $R^2$ und $R^3$ sind jeweils aus Wasserstoff, Alkyl, Aryl, Aralkyl und Alkaryl ausgewählt; $R^4$ ist aus Wasserstoff, Carboxy, N,N-Dialkylcarboxamido, Alkyl, Aryl, Aralkyl und Alkaryl ausgewählt; $R^5$ ist aus Alkyl, Aryl, Alkaryl und Aralkyl ausgewählt; und $Z^1$ ist der Rest eines vorgeformten Bildfarbstoffs, der zusammen mit der Gruppe —CO—NH— der Rest Z der farblosen Vorstufe eines vorgeformten Bildfarbstoffs ist.

29. Farblose, einen Farbstoff bildende Verbindung nach Anspruch 27, dadurch gekennzeichnet, daß die Verbindung M—Z die Formel

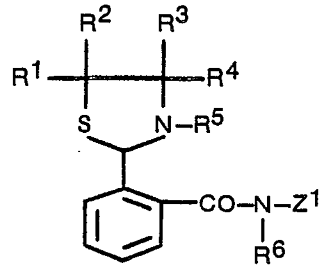

hat, worin bedeuten: $R^1$, $R^2$ und $R^3$ sind jeweils aus Wasserstoff, Alkyl, Aryl, Aralkyl und Alkaryl ausgewählt; $R^4$ ist aus Wasserstoff, Carboxy, N,N-Dialkylcarboxamido, Alkyl, Aryl, Aralkyl und Alkaryl ausgewählt; $R^5$ ist aus Alkyl, Aryl, Alkaryl und Aralkyl ausgewählt; $R^6$ ist Alkyl oder Aryl; und $Z^1$ ist der Rest eines vorgeformten Bildfarbstoffs, der zusammen mit der Gruppe

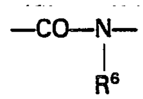

den Rest Z der farblosen Vorstufe eines vorgeformten Bildfarbstoffs darstellt.

30. Farblose, einen Farbstoff bildende Verbindung nach Anspruch 26, dadurch gekennzeichnet, daß die Verbindung M—Z die Formel

$$T^1—L^1—Y—Z^1$$

hat, worin bedeuten: $T^1$ ist eine Thiazolidin-2'-yl-Gruppe, die in der Lage ist, in Gegenwart von Silberionen und/oder einem löslichen Silberkomplex zu spalten; $L^1$ ist eine Gruppe, die in der Lage ist, eine β-Eliminierungsreaktion nach der Spaltung der Thiazolidin-2'-yl-Gruppe durchzuführen; Y ist eine austretende Gruppe, die bei der β-Eliminierungsreaktion freigesetzt wird; und $Z^1$ ist der Rest eines vorgeformten Bildfarbstoffs, der zusammen mit Y den Rest Z einer farblosen Vorstufe eines vorgeformten Bildfarbstoffs darstellt, wobei $T^1$—$L^1$— an dem Rest Z der Vorstufe substituiert ist, so daß die farblose Vorstufe in ihrer farblosen Form gehalten wird, bis die Thiazolidin-2'-yl-Gruppe der Spaltung unterliegt.

31. Farblose, einen Farbstoff bildende Verbindung nach Anspruch 30, dadurch gekennzeichnet, daß die Verbindung die Formel

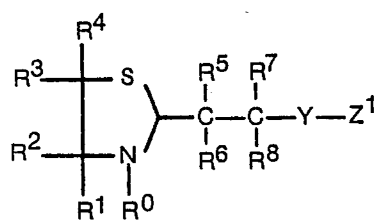

hat, worin bedeuten: $R^0$ ist aus Alkyl, Aryl, Aralkyl und Alkaryl ausgewählt; $R^1$ ist aus Wasserstoff, Carboxy, N,N-Dialkylcarboxamido, Alkyl, Aryl, Aralkyl und Alkaryl ausgewählt; $R^2$, $R^3$ und $R^4$ sind jeweils aus Wasserstoff, Alkyl, Aryl, Aralkyl und Alkaryl ausgewählt; $R^5$ ist aus Wasserstoff und einer Gruppe ausgewählt, die bei der Spaltung der Thiazolidinylgruppe unter Zurücklassung eines Elektronenpaars entfernt werden kann; $R^6$, $R^7$ und $R^8$ sind jeweils aus Wasserstoff, Alkyl, Aryl, Aralkyl und Alkaryl ausgewählt; und Y und $Z^1$ sind wie in Anspruch 30 definiert.

32. Farblose, einen Farbstoff bildende Verbindung nach Anspruch 26, dadurch gekennzeichnet, daß die Verbindung M—Z die Formel

$$T^1—L^1—Y^1—Z^1$$

hat, worin bedeuten: $T^1$ und $L^1$ sind wie in Anspruch 30 definiert; $Y^1$ ist eine Gruppe, die einer intramolekular beschleunigten nukleophilen Verdrängungsreaktion nach der β-Eliminierungsreaktion unterliegt; und $Z^1$ ist der Rest eines vorgeformten Bildfarbstoffs, der zusammen mit $Y^1$ den Rest Z einer

farblosen Vorstufe eines vorgeformten Bildfarbstoffs darstellt, wobei $T^1$—$L^1$— an dem Rest der farblosen Vorstufe substituiert ist, so daß die farblose Vorstufe M—Z in ihrer farblosen Form gehalten wird, bis die Thiazolidin-2'-yl-Gruppe der Spaltung unterliegt.

33. Farblose, einen Farbstoff bildende Verbindung nach Anspruch 32, dadurch gekennzeichnet, daß die Verbindung die Formel

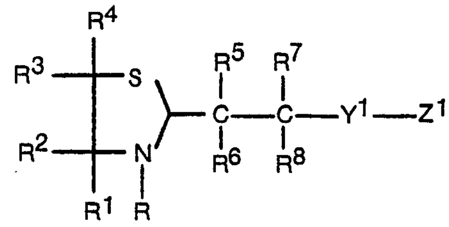

hat, worin bedeuten: R ist ausgewählt aus Alkyl, Aryl, Aralkyl und Alkaryl; $R^1$ ist ausgewählt aus Wasserstoff, Carboxy, N,N-Dialkylcarboxamido, Alkyl, Aryl, Aralkyl und Alkaryl; $R^2$, $R^3$ und $R^4$ sind jeweils ausgewählt aus Wasserstoff, Alkyl, Aryl, Aralkyl und Alkaryl; $R^5$ ist ausgewählt aus Wasserstoff und einer Gruppe, die bei der Spaltung der Thiazolidinylgruppe unter Hinterlassung eines Elektronenpaars entfernt werden kann, $R^6$, $R^7$ und $R^8$ sind jeweils ausgewählt aus Wasserstoff, Alkyl, Aryl, Aralkyl und Alkaryl; $Y^1$ und $Z^1$ sind wie in Anspruch 32 definiert.

**Revendications**

1. Produit photographique qui comprend une couche support portant, sur un de ses côtés, une couche d'halogènure d'argent sensible à la lumière et un précurseur incolore M—Z d'un colorant d'image préformé, ayant un fragment M et un radical Z, ledit fragment M comprenant un groupe A 1,3-soufre-azote possèdant un atome de carbone sur la position 2, intermédiaire entre lesdits atomes de soufre et d'azote, et subissant une scission en présence d'ions argent et/ou d'un complex soluble d'argent et ledit radical Z étant le radical du précurseur incolore du colorant d'image préformé, caractérisé en ce que ledit fragment M est substituant dudit radical Z de telle sorte que ledit précurseur incolore M—Z soit maintenu sous sa forme incolore jusqu'à ce que ledit groupe A 1,3-soufre-azote subisse la scission.

2. Produit photographique selon la revendication 1, caractérisé en ce que ledit précurseur incolore M—Z est non-diffusible dans une solution alcaline aqueuse.

3. Produit photographique selon la revendication 1 ou 2, caractérisé en ce que ledit colorant d'image fournit par ledit précurseur incolore M—Z est diffusible dans une solution alcaline aqueuse.

4. Produit photographique selon l'une quelconque des revendications précédentes, caractérisé en ce qu'il comprend en outre des moyens pour appliquer un liquide alcalin aqueux de développement contenant un agent de développement de l'halogènure d'argent et un solvant de l'halogénure d'argent.

5. Produit photographique selon la revendication 4, caractérisé en ce qu'au moins un dudit agent de développement et dudit solvant est contenu initialement dans une couche dudit produit photographique.

6. Produit photographique selon l'une quelconque des revendications précédentes, caractérisé en ce que ledit précurseur incolore M—Z est placé dans ladite couche d'halogènure d'argent.

7. Produit photographique selon l'une quelconque des revendications précédentes, caractérisé en ce que ledit halogènure d'argent fonctionne en négatif.

8. Produit photographique selon l'une quelconque des revendications précédentes, caractérisé en ce qu'il comprend une couche de réception de l'image adaptée pour fournir une image de transfert en couleur par diffusion du colorant sur celle-ci.

9. Produit photographique selon la revendication 8, caractérisé en ce qu'il comprend une couche réfléchissant la lumière entre lesdites couches d'halogènure d'argent et de réception de l'image, ce qui permet de regarder une image de transfert en couleur formée dans ladite couche de réception de l'image par réflexion, ladite couche réfléchissant la lumière étant efficace pour masquer la couche d'halogènure d'argent développée à une personne regardant ladite image en couleur.

10. Produit photographique selon l'une quelconque des revendications précédentes, caractérisé en ce qu'il comprend une couche à réaction acide.

11. Produit photographique selon la revendication 1, caractérisé en ce que ledit colorant d'image ne peut pas diffuser hors de la couche dans laquelle il est placé, au cours de l'exposition photographique.

12. Produit photographique selon l'une quelconque des revendications précédentes, caractérisé en ce que ledit groupe A 1,3-soufre-azote dudit fragment M dudit précurseur incolore M—Z possède un substituant amide L sur l'atome de carbone en position 2 qui, après la scission dudit groupe A 1,3-soufre-azote, subit une réaction de scission intramoléculairement accélérée.

13. Produit photographique selon la revendication 12, caractérisé en ce que ledit substituant L en position 2 dudit groupe A 1,3-soufre-azote est un substituant carboxamido.

14. Produit photographique selon la revendication 13, caractérisé en ce que ledit groupe A 1,3-soufre-azote est un groupe thiazolidin-2-yl.

15. Produit photographique selon la revendication 12, caractérisé en ce que ledit précurseur incolore M—Z possède la formule:

$$A—L—Z^1$$

dans laquelle A et L sont tels que définis dans la revendication 12, et $Z^1$ est un reste du colorant d'image préformé qui, lorsqu'il est pris avec L, forme le radical Z du précurseur incolore Z d'un colorant d'image préformé.

16. Produit photographique selon la revendication 15, caractérisé en ce que ledit précurseur incolore M—Z possède la formule

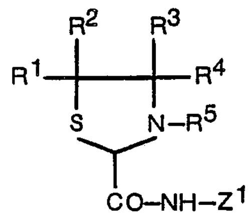

dans laquelle $R^1$, $R^2$ et $R^3$ sont chacun choisis parmi un atome d'hydrogène, un groupe alkyle, aryle, aralkyle et alkaryle; $R^4$ est choisi parmi un atome d'hydrogène, un groupe carboxyle, N-N-dialkylcarboxamido, alkyle, aryle, aralkyle et alkaryle; $R^5$ est choisi parmi un groupe alkyle, aryle, alkaryle et aralkyle; et $Z^1$ est un reste du colorant d'image préformé qui lorsqu'il est pris avec ledit —CO—NH—, est le radical Z du précurseur incolore Z d'un colorant d'image préformé.

17. Produit photographique selon la revendication 15, caractérisé en ce que ledit précurseur incolore M—Z a la formule:

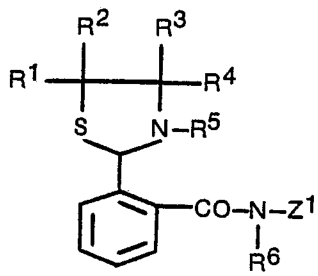

dans laquelle $R^1$, $R^2$ et $R^3$ sont chacun choisis parmi un atome d'hydrogène, un groupe alkyle, aryle, aralkyle et alkaryle; $R^4$ est choisi parmi un atome d'hydrogène, un groupe carboxyle, N,N-dialkylcarboxamido, alkyle, aryle, aralkyle et alkaryle; $R^5$ est choisi parmi un groupe alkyle, aryle, alkaryle et aralkyle; $R^6$ est un groupe alkyle ou aryle; et $Z^1$ est un reste du colorant d'image préformé, qui lorsqu'il est pris avec ledit

—CO—N—
  |
  $R^6$

est le radical Z du précurseur incolore d'un colorant d'image préformé.

18. Produit photographique selon l'une quelconque des revendications 1 à 11, caractérisé en ce que ledit groupe A 1,3-soufre-azote dudit fragment M dudit précurseur incolore M—Z est un groupe thiazolidinyle T possédant un substituant $L^1$ sur l'atome de carbone en position 2 qui, après scission dudit groupe thiazolidinyle, subit une réaction d'élimination en béta.

19. Produit photographique selon la revendication 18, caractérisé en ce que ladite réaction d'élimination en béta est suivie d'une réaction intramoléculairement accélérée de déplacement nucléophile.

20. Produit photographique selon la revendication 19, caractérisé en ce que ladite réaction d'élimination en béta réalise la scission d'un groupe amide.

21. Produit photographique selon la revendication 20, caractérisé en ce que ledit groupe amide est un groupe carboxamido.

22. Produit photographique selon la revendication 18, caractérisé en ce que ledit précurseur incolore M—Z a la formule

$$T^1—L^1—Y—Z^1$$

dans laquelle $T^1$ est un groupe thiazolidin-2'-yl capable de subir une scission en présence d'ions argent et/

ou d'un complexe soluble d'argent; $L^1$ est tel que défini dans la revendication 18; Y est un groupe partant libéré par ladite réaction d'élimination en béta; et $Z^1$ est un reste du colorant d'image préformé qui lorsqu'il est pris avec Y est le radical Z du précurseur incolore dudit colorant d'image préformé, ledit $T^1$—$L^1$— étant substituant dudit radical Z du précurseur de telle sorte que le précurseur incolore M—Z soit maintenu dans sa forme incolore jusqu'à ce que ledit groupe thiazolidin-2'-yl subisse ladite scission.

23. Produit photographique selon la revendication 22, caractérisé en ce que ledit précurseur incolore M—Z possède la formule

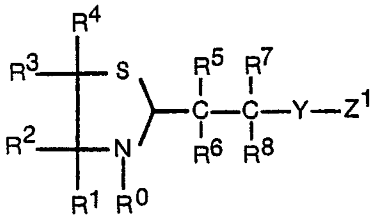

dans laquelle $R^0$ est choisi parmi un groupe alkyle, aryle, aralkyle et alkaryle; $R^1$ est choisi parmi un atome d'hydrogène, un groupe carboxyle, N,N-dialkylcarboxamido, alkyle, aryle, aralkyle et alkaryle; $R^2$, $R^3$ et $R^4$ sont chacun choisi parmi un atome d'hydrogène, un groupe alkyle, aryle, aralkyle et alkaryle; $R^5$ est choisi parmi un atome d'hydrogène et un groupe qui peut être éliminé après scission dudit groupe thiazolidinyle pour laisser une paire d'électrons; $R^6$, $R^7$ et $R^8$ sont chacun choisi parmi un atome d'hydrogène, un groupe alkyle, aryle, aralkyle et alkaryle; et Y est tel que défini dans la revendication 22; et $Z^1$ est un reste du colorant d'image préformé qui, lorsqu'il est pris avec Y est le radical Z du précurseur incolore d'un colorant d'image préformé.

24. Produit photographique selon la revendication 19, caractérisé en ce que ledit précurseur incolore M—Z a la formule:

$$T^1—L^1—Y^1—Z^1$$

dans laquelle $T^1$ est un groupe thiazolidin-2'-yl capable de subir une scission en présence d'ions argent et/ou d'un complexe soluble d'argent; $L^1$ est tel que défini dans la revendication 18; $Y^1$ est un fragment capable de subir une réaction intramoléculairement accélérée de déplacement nucléophile après ladite réaction d'élimination en béta; et $Z^1$ est un reste du colorant d'image préformé qui, lorsqu'il est pris avec $Y^1$, est le radical Z du précurseur incolore d'un colorant d'image préformé, ledit $T^1$—$L^1$— étant substituant dudit radical du précurseur incolore de telle sorte que le précurseur incolore M—Z soit maintenu dans sa forme incolore jusqu'à ce que ledit groupe thiazolidin-2'-yl subisse ladite scission.

25. Procédé photographique qui comprend les étapes de photoexposition d'un produit photographique comprenant une émulsion d'halogènure d'argent avec laquelle est associée un précurseur incolore d'un colorant d'image préformé; et de développement de ladite émulsion exposée d'halogènure d'argent pour former, selon une fonction du développement, une image en couleur en le colorant provenant dudit précurseur incolore; caractérisé en ce que ledit produit photographique est un produit selon l'une quelconque des revendications précédentes.

26. Composé incolore fournisseur de colorant, répondant à la formule

$$M—Z$$

dans laquelle M est un fragment comprenant un groupe A 1,3-soufre-azote possèdant un atome de carbone dans la position 2 intermédiaire entre lesdits atomes de soufre et d'azote, et subissant une scission en présence d'ions argent et/ou d'un complexe soluble d'argent et Z est le radical d'un précurseur incolore d'un colorant d'image préformé, caractérisé en ce que ledit fragment M est substituant dudit radical Z de telle sorte que ledit précurseur incolore soit maintenu dans sa forme incolore jusqu'à ce que ledit groupe A 1,3-soufre-azote subisse la scission.

27. Composé incolore fournissant le colorant selon la revendication 26, caractérisé en ce que ledit composé M—Z possède la formule

$$A—L—Z^1$$

dans laquelle A est tel que défini dans la revendication 26; L est un fragment amide capable de subir une réaction de scission intramoléculairement accélérée après scission dudit groupe 1,3-soufre-azote; et $Z^1$ est un reste de colorant d'image préformé qui, lorsqu'il est pris avec L, est le radical Z du précurseur incolore d'un colorant d'image préformé.

28. Composé incolore fournisseur de colorant selon la revendication 27, caractérisé en ce que ledit composé possède la formule:

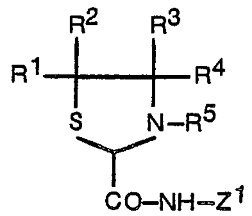

dans laquelle $R^1$, $R^2$ et $R^3$ sont chacun choisis parmiun atome d'hydrogène, un groupe alkyle, aryle, aralkyle et alkaryle; $R^4$ est choisi parmi un atome d'hydrogène, un groupe carboxyle, N,N-dialkylcarboxamido, alkyle, aryle, aralkyle et alkaryle; $R^5$ est choisi parmi un groupe alkyle, aryle, alkaryle et aralkyle; et $Z^1$ est un reste du colorant d'image préformé qui, lorsqu'il est pris avec ledit —CO—NH—, est le radical Z du précurseur incolore d'un colorant d'image préformé.

29. Composé incolore fournisseur de colorant selon la revendication 27, caractérisé en ce que ledit composé M—Z possède la formule

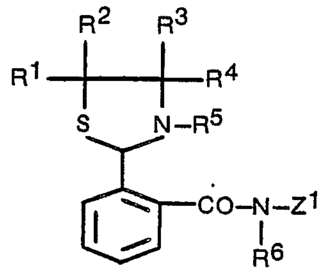

dans laquelle $R^1$, $R^2$ et $R^3$ sont chacun choisis parmi un atome d'hydrogène, un groupe alkyle, aryle, aralkyle et alkaryle; $R^4$ est choisi parmi un atome d'hydrogène, un groupe carboxyle, N,N-dialkylcarboxamido, alkyle, aryle, aralkyle et alkaryle; $R^5$ est choisi parmi un groupe alkyle, aryle, alkaryle et aralkyle; $R^6$ est un groupe alkyle ou aryle; et $Z^1$ est un reste d'un colorant d'image préformé qui, lorsqu'il est pris avec le dit

$$-CO-\underset{\substack{|\\R^6}}{N}-,$$

est le radical Z du précurseur incolore d'un colorant d'image préformé.

30. Composé incolore fournisseur de colorant selon la revendication 26, caractérisé en ce que ledit composé M—Z a la formule

$$T^1-L^1-Y-Z^1$$

dans laquelle $T^1$ est un groupe thiazolidin-2'-yl capable de subir une scission en présence d'ions argent et/ou d'un complexe soluble d'argent; $L^1$ est un fragment capable de subir une réaction d'élimination en béta après la scission dudit groupe thiazolidin-2'-yl; Y est un groupe partant libéré par ladite réaction d'élimination en béta; et $Z^1$ est un reste du colorant d'image préformé qui, lorsqu'il est pris avec Y, est le radical Z du précurseur incolore dudit colorant d'image préformé, ledit $T^1$—$L^1$— étant substituant dudit radical Z du précurseur de telle sorte que le précurseur incolore soit maintenu dans sa forme incolore jusqu'a ce que ledit groupe thiazolidin-2'-yl subisse ladite scission.

31. Composé incolore fournisseur de colorant selon la revendication 30, caractérisé en ce que ledit composé a la formule

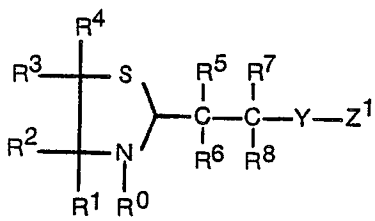

dans laquelle $R^0$ est choisi parmi un groupe alkyle, aryle, aralkyle et alkaryle; $R^1$ est choisi parmi un atome d'hydrogène, un groupe carboxyle, N,N-dialkylcarboxamido, alkyle, aryle, aralkyle et alkaryle; $R^2$, $R^3$ et $R^4$

sont chacun choisis parmi un atome d'hydrogène, un groupe alkyle, aryle, aralkyle et alkaryle; $R^5$ est chosi parmi un atome d'hydrogène et un groupe qui peut être éliminé après la scission dudit groupe thiazolidinyle pour laisser une paire d'électrons; $R^6$, $R^7$ et $R^8$ sont chacun choisis parmi un atome d'hydrogène, un groupe alkyle, aryle, aralkyle et alkaryle; et Y et $Z^1$ sont tels que définis dans la revendication 30.

32. Composé incolore fournisseur de colorant selon la revendication 26, caractérisé en ce que ledit composé M—Z a la formule

$$T^1—L^1—Y^1—Z^1$$

dans laquelle $T^1$ et $L^1$ sont tels que définis dans la revendication 30; $Y^1$ est un fragment capable de subir une réaction intramoléculairement accélérée de déplacement nucléophile après ladite réaction d'élimination en béta; et $Z^1$ est un reste du colorant d'image préformé qui, lorsqu'il est pris avec $Y^1$, est le radical Z du précurseur incolore d'un colorant d'image préformé, ledit $T^1$—$L^1$— étant substituant dudit radical du précurseur incolore de telle sorte que le précurseur incolore M—Z soit maintenu dans sa forme incolore jusqu'à ce que ledit groupe thiazolidin-2'-yl subisse ladite scission.

33. Composé incolore fournisseur de colorant selon la revendication 32, caractérisé en ce que ledit composé a la formule

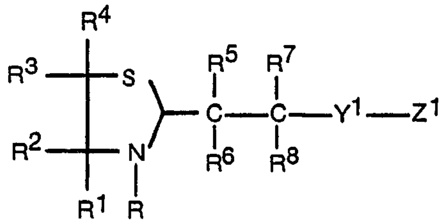

dans laquelle R est choisi parmi un groupe alkyle, aryle, aralkyle et alkaryle;, $R^1$ est choisi parmi un atome d'hydrogène, un groupe carboxyle, N,N-dialkylcarboxamido, alkyle, aryle, aralkyle et alkaryle; $R^2$, $R^3$ et $R^4$ sont chacun choisi parmi un atome d'hydrogène, un groupe alkyle, aryle, aralkyle et alkaryle; $R^5$ est choisi parmi un atome d'hydrogène et un groupe qui peut être éliminé après la scission dudit groupe thiazolidinyle pour laisser une paire d'électrons; $R^6$, $R^7$ et $R^8$ sont chacun choisis parmi un atome d'hydrogène, un groupe alkyle, aryle, aralkyle et alkaryle; $Y^1$ et $Z^1$ sont tels que définis dans la revendication 32.